Europäisches Patentamt

European Patent Office

Office européen des brevets

(11) Publication number: **0 284 044 B1**

(12) **EUROPEAN PATENT SPECIFICATION**

(45) Date of publication of patent specification: 23.03.94 (51) Int. Cl.5: **C12N 15/67**, C12N 15/81, C12N 9/10

(21) Application number: **88104649.4**

(22) Date of filing: **23.03.88**

(54) **High level expression in yeast.**

(30) Priority: **23.03.87 US 29867**

(43) Date of publication of application:
**28.09.88 Bulletin 88/39**

(45) Publication of the grant of the patent:
**23.03.94 Bulletin 94/12**

(84) Designated Contracting States:
**AT BE CH DE FR GB IT LI LU NL SE**

(56) References cited:
**EP-A- 0 171 142**
**WO-A-87/03008**

**NATURE, vol. 304, 18th August 1983, pages 652-654, Macmillan Journals Ltd,London, GB; D.W. RUSSELL et al.: "DNA sequences of two yeast promoter-upmutants"**

(73) Proprietor: **ZYMOGENETICS, INC.**
**4225 Roosevelt Way N.E.**
**Seattle, WA 98108(US)**

(72) Inventor: **Irani, Meher H.**
**3503 N.E. 100th**
**Seattle**
**Washington 98125(US)**
Inventor: **Kilgore, Tammy L.**
**3941-1st Avenue, N.E.**
**204 Seattle Washington 98115(US)**

(74) Representative: **Brown, John David et al**
**FORRESTER & BOEHMERT**
**Franz-Joseph-Strasse 38**
**D-80801 München (DE)**

Rank Xerox (UK) Business Services
(3.10/3.09/3.3.3)

**Description**

Cross-Reference to Related Application

This application is a continuation-in-part to U.S. Patent Application Serial No. 029,867, filed March 23, 1987, which application is pending.

Technical Field

The present invention relates to a method for the enhanced production of proteins in yeast host cells in general, and more specifically, to an improved method for the increased production to α-1-antitrypsin and other proteins.

Background Art

Current technology in the field of genetic engineering has facilitated the expression of foreign genes in yeast. The production of eukaryotic (e.g., mammalian) gene products in yeast has advantages over production using mammalian or bacterial cell culture. One of the major disadvantages in the use of bacteria as a host for the production of heterologous proteins is the production of endotoxins which must be completely removed before the product can be used as a pharmaceutical agent. Heterologous proteins produced in bacteria have been shown to have low solubility, a problem which, unless overcome, severely limits their use as pharmaceuticals. Further, the use of mammalian cells to express a protein product at commercial levels is much more expensive. In contrast, commercial scale fermentation of yeast is well established, allowing for the production of large quantities of heterologous protein products. Yeast is a eukaryotic organism that shares greater similarity with mammalian cells than do bacteria.

Recent concern with the use of human fluid-derived products, and the difficulty and expense that accompany the purification of such proteins, have provided an interest in the production of recombinant proteins in yeast. An example of a protein which has been expressed in yeast is alpha-1-antitrypsin (AAT) (Kawasaki, U.S. Patent No. 4,599,311, and Kawasaki et al., U.S. Patent No. 4,711,848).

The protease inhibitor, AAT, also known as α-1 protease inhibitor, is a component of mammalian blood that acts as an inhibitor of proteolytic enzymes, such as serine proteases. In mammals, the major physiological function of α-1-antitrypsin is the inhibition of elastase, a potent protease that hydrolyzes structural proteins. Elastase acts to assist in the degradation of inhaled particulate matter in the lungs. The inhibitory activity exhibited by α-1-antitrypsin is hypothesized to be a defensive, selfregulatory mechanism that protects the body's tissues from attack by elastase and by other proteolytic enzymes released during an inflammatory response.

Genetic deficiencies that result in reduced levels of α-1-antitrypsin have been indentified in humans. Genetic variants, designated Z and S, are mutant alleles that produce reduced levels of α-1-antitrypsin in individuals that carry combinations of these alleles (e.g., SS, SZ). These genetic deficiencies may be accompanied by degenerative lung diseases and, in some cases, by liver disease. The low levels of α-1-antitrypsin activity associated with the mutant alleles may be further reduced through the inactivating oxidation of α-1-antitrypsin by environmental pollutants, including tobacco smoke. It has also been postulated that emphysema in smokers who are homozygous for the normal allele is the result of smoke-induced oxidation of normal α-1-antitrypsin.

Replacement therapy has been used successfully to treat patients with α-1-antitrypsin deficiency (Gadek et al., J. Clin. Invest. 68:1158-1165, 1981). The administered α-1-antitrypsin, concentrated from pooled human hepatitis-free donor plasma, established anti-elastase activity within alveolar structures and produced no untoward side effects.

The expression of α-1-antitrypsin and other heterologous proteins in yeast has generally resulted in expression levels ranging from about 1% to 5% of total protein. In contrast, yeast proteins encoded by genes present on multicopy plasmids may be produced at levels as high as 50% to 80% of total protein expressed (Mellor et al., Gene 33:215-226, 1985). It therefore appears that expression levels of heterologous DNA sequences in yeast may be regulated, at least in part, by the nature of the heterologous sequence itself and by the turnover rate of the encoded protein (Mellor et al., ibid.). Mellor et al. have suggested that foreign proteins are unstable in yeast, and that steady state levels of heterologous mRNA are relatively low. On the other hand, Kramer et al. (Proc. Natl. Acad. Sci. USA 81:367-370, 1984) and Kniskern et al. (Gene 46:135-141, 1986) have shown that steady state levels of heterologous mRNA are high relative to the amount of heterologous protein expressed, and in the case of hepatitis B core antigen, the heterologous protein is more stable than yeast proteins.

Other factors may also influence yeast expression levels of heterologous genes, but manipulation of these factors has not generally increased heterologous protein expression levels above approximately 5% of total protein expression. Even when these factors are optimized, the expression levels of foreign genes do not approach the levels obtained with cloned yeast genes.

Several examples of low level expression of foreign genes in yeast have appeared in the literature. Alpha interferon, for instance, has been expressed in yeast by many investigators using a variety of promoters. Mellor et al. (ibid.), Tuite et al. (EMBO J. 1:603-608, 1982) and Hitzeman et al. (Science 219:620-625, 1983) used the yeast phosphoglycerate kinase (PGK1) gene promoter to produce alpha interferon at levels between 1% and 3% of total cell protein. In each case, the PGK1 promoter-alpha interferon expression unit was used in vectors derived from the yeast 2 micron plasmid. The constitutive promoter from the yeast ADH1 gene was used by Hitzeman et al. (Nature 293:717-722, 1981) to express alpha interferon in the yeast vector YRp7. These constructions yielded alpha interferon at 0.4% of total cell protein. Bitter and Egan (Gene 32:263-274, 1983) used the glyceraldehyde-3-phosphate dehydrogenase (GAP491, also known as GPD) gene promoter to express alpha interferon in vectors derived from the yeast 2 micron plasmid. These constructs yielded alpha interferon at approximately 1% total cell protein. Kramer et al. (Proc. Natl. Acad. Sci. USA 81:367-370, 1984) used the yeast repressible acid phosphatase (PHO5) gene promoter to express alpha interferon on a vector derived from the yeast 2 micron plasmid. Upon induction, this construction yielded alpha interferon at levels of 0.2% of total cell protein.

Other proteins that have been expressed in yeast at levels comparable to alpha interferon are hepatitis B surface antigen (HBS) and human $\alpha$-1-antitrypsin (AAT). Bitter and Egan (ibid.) have used the GAP491 promoter to express HBS at levels up to 4% of total cell protein. Using the PHO5 promoter, as much as 3.4 ug of HBS per ml of yeast mid-log phase culture ($1.5 \times 10^7$ cells/ml) was obtained (Miyanohara et al., Proc. Natl. Acad. Sci. USA 80:1-5, 1983). Assuming that $10^{10}$ cells contain about 120 mg total protein (Robert A. Smith, personal communication), a yield of about 2 percent HBS can be calculated. Valenzuela et al. (Nature 298:347-350, 1982) reported that HBS expression using the ADH1 promoter resulted in expression levels approximately two orders of magnitude lower than those reported by Miyanohara et al. (ibid.).

The expression of AAT has produced similarly low levels of expression. The TPI1 promoter was used to produce AAT levels of approximately 0.9% of total cell protein (Kawasaki, U.S. Patent No. 4,599,311, 1986). A high copy number plasmid has been used to obtain AAT levels of 4% to 6% of total cell protein (Kawasaki and Bell, EP 171, 142, published 1986). Other investigators have achieved AAT expression levels of 1.2% (Cabezon, EP 151,102, 1985) and 1% (Rosenberg et al., Nature 312:77-80, 1984).

There are certain exceptions to these examples of low heterologous protein expression in yeast. These include superoxide dismutase (SOD) and hepatitis B core antigen (HBcAg). Hallewell and Mullenbach (WO85/01503, 1985), have disclosed the use of the GAP491 promoter to produce SOD at a reported level of 14.8% total cell protein. Kniskern et al. (Gene 46:135-141, 1986) have also used the GAP491 promoter to produce hepatitis B core antigen at 40% total cell protein. Kniskern et al. (ibid.) note that the high level of HBcAg may be related to the unusual stability of the heterologous protein which forms aggregates within the cell, and could protect the protein from endoproteolytic attack. Cousens et al. (EP 196,056) disclose a method of increasing expression levels of heterologous proteins by producing fusion proteins which may be subsequently cleaved in vitro.

In summary, heterologous proteins are generally produced at low levels (less than ⁻5% of total cell protein) in yeast. Although the reasons for this phenomenon are not well understood, it has been postulated that the nature of the foreign proteins themselves and/or low levels of foreign mRNAs limit expression levels. HBcAg, a notable exception, appears to be an unusual protein whose structure enhances its production level. This exception lends further support to the hypothesis that expression levels may be protein dependent.

Consequently, there exists a need in the art for a method of enhancing the production of heterologous proteins, such as $\alpha$-1-antitrypsin, in yeast host cells. The present invention fulfills this need and further provides other related advantages.

Disclosure of the Invention

Briefly stated, the present invention discloses an expression vector capable of directing the expression of heterologous genes or cDNA in yeast, the expression vector containing an ADH2 promoter, a heterologous gene or cDNA, and a defective selectable marker. In one preferred embodiment, the heterologous gene or cDNA encodes $\alpha$-1-antitrypsin.

Another aspect of the present invention discloses a yeast host cell having a genetic defect, into which has been introduced an expression vector capable of directing the expression of heterologous genes or cDNAs in yeast, the expression vector containing an ADH2 promoter, a heterologous gene or cDNA, and a defective selectable marker, the marker complementing the genetic defect in the yeast host cell.

The present invention further provides a method for the enhanced production of proteins in yeast host cells. The method includes (a) introducing into a yeast host cell having a genetic defect, an expression vector capable of directing the expression of heterologous genes or cDNA in yeast, the expression vector containing an ADH2 promoter, a heterologous gene or cDNA, and a defective selectable marker, the defective selectable marker complementing the genetic defect in the yeast host cell; (b) growing the yeast host cell in an appropriate medium; and (c) isolating the protein produced by the host cell.

In one preferred embodiment of the invention, the yeast host cell is a [cir°] strain of S. cerevisiae auxotrophic for growth on leucine and carrying a pep4 mutation which results in reduced activity of several vacuolar proteases; the heterologous gene encodes $\alpha$-1-antitrypsin; and the expression vector includes an origin of replication, REP1, REP2 and REP3 genes derived from a yeast 2 micron circle, an ADH2-4$^c$ promoter, and a defective selectable marker consisting of the leu2-d gene. In a particular embodiment, the $\alpha$-1-antitrypsin is retained substantially within the cytoplasm of the host cell, and the yeast host cell is disrupted prior to isolating the $\alpha$-1-antitrypsin produced by the yeast cells.

Other aspects of the invention will become evident upon reference to the following detailed description and attached drawings.

## Brief Description of the Drawings

Figure 1 illustrates the subcloning of a cDNA sequence encoding alpha-1-antitrypsin.

Figure 2 illustrates the construction of plasmid pAT-1.

Figure 3 illustrates the construction of a plasmid comprising the ADH2-4$^c$ promoter fused to the first amino acid codon of the mature alpha-1-antitrypsin cDNA sequence.

Figure 4 illustrates the construction of plasmids comprising ADH2 promoter sequences.

Figure 5 illustrates the construction of the alpha-1-antitrypsin expression vector pAT-3.

Figure 6 illustrates the construction of expression vectors pAT-4 and pAT-6.

## Best Mode for Carrying Out the Invention

Prior to setting forth the invention, it may be helpful to an understanding thereof to set forth definitions of certain terms to be used hereinafter.

Defective selectable marker: A gene or cDNA that is expressed at a low specific activity and is used on a plasmid to allow for selection of cells transformed with the plasmid. Such a gene may be expressed at a low rate due to deletions, mutations or alterations of a promoter region. An example of such a gene is the leu2-d gene isolated by Beggs (Nature 275:104-108, 1978). The low rate of expression may also be caused by the use of a heterologous promoter to drive the expression of the selectable marker. An example of this type of selectable marker is the E. coli lacZ promoter used to express the Aspergillus nidulans tpiA cDNA in the yeast S. cerevisiae (described herein). Alternatively, a heterologous gene may be used in which the expression rate is low due to altered transcription sites, as in the case of the Schizosaccharomyces pombe POT1 gene (P. R. Russell, Gene 40:125-130, 1985). The defective selectable marker may also encode a protein with a low specific activity. This gene may be an altered homologous gene encoding a protein with a reduced specific activity. Alternatively, a heterologous gene may be used that only weakly complements a defect in the host.

Genetic defect: A lesion within a specific gene in a host cell resulting from a point mutation, deletion, or disruption and resulting in the Mendelian segregation of the defect in the progeny of a cross. Such a deficiency may lead, for example, to an auxotrophic condition with regards to specific nutrients. An example of this sort of lesion is the leu2-3,112 mutation in S. cerevisiae, which leads to a requirement for leucine in the growth media in these mutants. Alternatively, the lesion may result in the inability of an organism containing the lesion to utilize a specific carbon source. Such an example is the tpiI mutation in S. cerevisiae, which prevents mutants from growing on media that contain glucose as the sole carbon source. Genetic defects maya generally be complemented by plasmid-borne selectable markers.

2 micron circle: A naturally occurring yeast plasmid circle capable of overriding cell-cycle control of replication to achieve and maintain up to 60-100 copies per haploid genome. The 2 micron plasmid circle can interconvert between two distinct forms by autocatalyzed intramolecular recombination. The plasmid contains its own origin of replication and genes designated REP1, REP2 and REP3, which are involved in maintenance and replication of the plasmid. Replication and recombination of the 2 micron circle are discussed by Broach and Hicks (Cell 21:501-508, 1980).

[Cir °] strains: Yeast strains which do not contain the 2 micron plasmid.

Mutant isolate: A DNA sequence differing from the wild-type allele. Such a difference may result in an altered phenotype in a cell carrying the mutant sequence. For example, the wild-type ADH2 promoter of S. cerevisiae is strongly repressed in cells grown on glucose as the carbon source, and is depressed in cells grown on ethanol. Several mutant isolates, designated ADH2$^c$ or ADR3$^c$, have been described which exhibit altered activity under depression conditions. As used herein, the term "mutant isolate of the ADH2 promoter" includes, but is not limited to, these particular isolates. The term "ADH2 promoter" will include both the wild-type promoter and mutant isolates thereof. These promoters will comprise at least the regulatory, RNA polymerase binding, and transcription initiation functions of the wild-type promoter.

As noted above, a number of researchers have achieved low level expression of a variety of heterologous proteins in yeast. One such protein, α-1-antitrypsin (AAT), has heretofore been expressed only at levels of about 1% to 6%. The present invention is based on the inventors' discovery that a yeast ADH2 promoter, when used in conjunction with a defective selectable marker, is capable of directing high levels of expression of heterologous genes or cDNA in yeast host cells. The expression vectors disclosed within the present invention are stable and are capable of autonomous replication to high copy number in yeast host cells. The inclusion of a regulated ADH2 promoter allows the yeast host cells to be grown to high density under conditions that repress the transcription of the heterologous mRNA. Once a predetermined cell density has been achieved, expression of the foreign gene or cDNA can then be derepressed, or switched "on." In a preferred embodiment, the yeast expression system disclosed herein produces levels of AAT expression that constitute up to 30% or more of total cell protein.

Given that few heterologous proteins have been expressed in yeast at levels greater than about 5% of total protein, and that expression levels may be limited by the nature of the heterologous protein or mRNA, it would not be expected that the use of an ADH2 promoter in combination with a defective selectable marker would increase expression levels by at least 5- to 6-fold.

As noted above, the present invention discloses an expression vector containing an ADH2 promoter, a heterologous gene or cDNA, and a defective selectable marker. In a preferred embodiment, the expression vector includes DNA sequences of the endogenous yeast 2 micron plasmid. A particularly preferred expression vector includes the 2 micron circle plasmid origin of replication and the 2 micron REP1, REP2 and REP3 genes. In another preferred embodiment, the expression vector of the present invention contains a transcriptional terminator. A particularly preferred transcription terminator is derived from the TPI1 gene.

Preferred ADH2 promoters include the ADH2$^c$ promoters (Ciriacy, Mutat. Res. 29:315-326, 1975; Ciriacy, Molec. Gen. Genet. 145:327-333, 1976; Ciriacy, Molec. Gen. Genet. 176:427-431, 1979; Williamson and Young, Cell 23:605-614; 1981). A particularly preferred ADH2$^c$ promoter is the ADH2-4$^c$ promoter. As previously noted, the ADH2 promoter contains sequences responsible for regulation of transcription, RNA polymerase binding, and initiation of transcription. In the wild-type ADH2 promoter, regulatory function is associated with the region of dyad symmetry extending from about nucleotide -292 to about nucleotide -271; RNA polymerase binding is associated with the TATA box at around nucleotide -160; and transcription initiation occurs in the region of about -60 to -50.

A defective selectable marker, as used herein, allows only low level complementation for a genetic defect in a yeast host strain. The low level of complementation in turn produces a compensating increase in copy number of the expression vector, a requisite for growth of the host cell under selective conditions (Erhart and Hollenberg, J. Bacteriol. 156:625, 1983). In a preferred embodiment of the present invention, the defective selectable marker comprises a gene or cDNA that encodes a protein expressed at a low level due to the use of a heterologous promoter or due to altered transcription start sites. The Aspergillus nidulans tpiA cDNA and the Schizosaccharomyces pombe POT1 gene, respectively, are particularly preferred in this regard. Within an alternate preferred embodiment, the defective selectable marker includes a deletion or alteration in a promoter operatively linked to the marker. A particularly preferred defective selectable marker in this regard is the leu2-d gene.

It will be evident to one skilled in the art that, in addition to a heterologous gene or cDNA that encodes α-1-antitrypsin, a variety of other heterologous genes or cDNAs may be employed within the present invention.

One example of a protein that can be expressed at high levels in yeast is the coagulation factor, Factor XIII. Factor XIII is a component of the blood coagulation system, which when activated by thrombin, acts in the final stages of blood coagulation to cross-link fibrin polymers to stabilize and strengthen the fibrin clot. Factor XIII, which consists of a tetramer of two a subunits and two b subunits, has been cloned as two cDNAs, one encoding the a subunit and the other encoding the b subunit (Ichinose et al., Biochem. J.

25:6900-6906, 1986 and Ichinose et al., Biochem. J. 25:4633-4638, 1986, respectively). The a subunit cDNA has been expressed in yeast (as described in copending commonly assigned U.S. Patent Application Serial No. 909,512 which is incorporated by reference herein in its entirety) using an expression unit driven by an ADH2 promoter and inserted in the expression vectors described herein. Transformation of the expression vectors into the appropriate yeast strain has demonstrated that the expression vectors of the invention yield up to 25 times more Factor XIII activity than an expression vector utilizing the conventional URA3 selectable marker.

Another example of a protein that can be expressed in yeast is the anticoagulant PAP-I (Funakoshi et al., Biochem J. 26:5572-5578, 1987). PAP-I is a protein which has been tentatively grouped with the family of lipocortins and exhibits the ability to bind to phospholipid. A cDNA has been cloned which encodes the PAP-I protein (described in copending U.S. Patent Application Serial Nos. 011,782 and 059,355, which are incorporated by reference herein in their entirety). This cDNA has been used in an expression vector, described herein, and has been transformed into an appropriate yeast strain to produce PAP-I. In a preferred embodiment, the yeast expression system disclosed herein produces levels of PAP-I expression which are 10 times the levels found using an expression vector utilizing the conventional LEU2 selectable marker.

The expression vectors of the present invention may be introduced into a yeast host cell that has one or more genetic defects, with the defective selectable marker(s) of the expression vector complementing the genetic defect(s) in the yeast host cell. Suitable host strains may be obtained from depositories such as American Type Culture Collection, Rockville, Maryland, and the Yeast Genetic Stock Center, Berkeley, California, or may be prepared using standard mutagenesis techniques. Procedures for introducing an expression vector into a yeast host cell are well known in the literature (for example, Beggs, ibid., 1978), and Hinnen et al., Proc. Natl. Acad. Sci. USA 75:1929-1933, 1978). Suitable host cells in this regard include strains of Saccharomyces cerevisiae, Saccharomyces douglasii and Saccharomyces carlsbergenis, with [cir°] strains particularly preferred. Other species of yeast known to carry endogenous 2 micron plasmid can be cured to a [cir°] state (e.g., by the method of Dobson et al., Curr. Genet. 2:210-215, 1980). Saccharomyces douglasii is a heterothallic yeast initially isolated by Donald C. Hawthorne. S. douglasii strain 4770-1A has been successfully transformed with the vector YEp13, indicating that the strain contains endogenous 2 micron plasmid capable of complementing the replication functions missing from the YEp13 vector. Saccharomyces carlsbergensis is another species known to carry endogenous 2 micron plasmid. Strains of S. carlsbergensis have been isolated and studied by Guerineu et al. (Biochem. Biophys. Res. Commun. 61:462, 1974) and Livingston (Genetics 86:73, 1977). In a preferred embodiment, the yeast host strain carries a pep4 mutation which results in reduced activity of several vacuolar proteases that could otherwise degrade a heterologous protein produced in the host strain.

Transformed yeast host cells are obtained by selecting for complementation of the genetic defect by the selectable marker present on the plasmid. The selected yeast host cells are grown in an appropriate medium. Generally, the cells are grown overnight in selective medium containing glucose, then switched to ethanol-containing medium to derepress foreign gene or cDNA expression. Alternatively, the cells are grown in selective medium containing glucose and the cells are allowed to exhaust the glucose which will cause the depression of the foreign gene or cDNA expression. The expressed heterologous protein produced by the transformed host cells is then isolated according to conventional procedures.

Methods for purification of heterologous proteins produced in transformed yeast cells are generally known in the art. Where the protein is retained within the host cell, it will be necessary to first disrupt the cell and remove cell debris, preferably by centrifugation, to produce a cleared lysate. In the case of a secreted protein, the protein is purified directly from the culture medium. The cleared lysate or medium is then fractionated by conventional protein purification methods. A multi-step process will generally be used. Typical procedures in this regard include precipitation (e.g., with polyethylene glycol or ammonium sulfate), ion exchange chromatography, affinity chromatography, preparative gel electrophoresis, high performance liquid chromatography, and fast pressure liquid chromatography. In many cases it is preferable to concentrate the fractions of interest between steps, such as by ammonium sulfate precipitation followed by dialysis to remove excess salt. The selection and ordering of the various steps will depend on the characteristics of the particular protein of interest, and is within the level of ordinary skill in the art.

In a typical preparation, yeast host cells producing a heterologous protein, such as AAT, are harvested by centrifugation and are resuspended in a suitable buffer, such as phosphate-buffered saline (PBS). The cells are disrupted, preferably by vortexing with glass beads. It is also preferable to remove unwanted proteins from the disrupted cell-glass bead slurry, such as by precipitation with 15% Polyethylene Glycol MW1000 (PEG-1000). The mixture is then centrifuged to remove glass beads and nonsolubilized cell material. To monitor purification, the supernatant may be assayed for protein content using standard procedures, such as the method of Lowry et al. (J. Biol. Chem. 193:265-275, 1951), and for the protein of interest, such as by gel electrophoresis or activity assay. The supernatant is then applied to an ion exchange column, such as DEAE- Sephacel Fast Flow (Pharmacia, equilibrated with 50 mM Tris, pH 6.5). Protein is eluted by stepwise application of buffer containing varying concentrations of NaCl. AAT typically elutes at 0.2 M NaCl. Fractions containing AAT are dialyzed (e.g., in 50 mM Tris pH 8). The dialyzed material is then applied to an ion exchange column, preferably Pharmacia Mono-Q, on a Fast Pressure Liquid Chromatograph (FPLC, Pharmacia, Piscataway, N.J.). Fractions are collected and may be visualized by polyacrylamide gel electrophoresis.

The protein may be further purified by pooling the peak fractions from FPLC and precipitating the protein with 70% ammonium sulfate. The precipitate is harvested by centrifugation and resuspended, preferably in 0.2 M sodium phosphate buffer, pH 7.2. The redissolved precipitate is then size separated using liquid chromatography. Collected fractions may be visualized by polyacrylamide gel electrophoresis. Peak fractions are pooled and frozen at -80°C.

Yields of AAT may be assayed using a "sandwich type" enzyme linked immunosorbent assay (ELISA), using a monoclonal antibody to AAT and a rabbit antiserum generated against human AAT that has been affinity purified and coupled to biotin. The anti-AAT monoclonal antibody is typically plated out in standard 96 well miocrotiter plates. After absorption of the anti-AAT antibody, the plates are blocked with buffer containing bovine serum albumin. After the blocking buffer is removed, AAT samples are added to the plates in triplicate. A series of purified AAT standards (>95% pure, as determined by electrophoresis) are also included on each 96-well plate. After incubation and washing, the detecting antibody-biotin conjugate is added to the plate and after another incubation and washing, avidin-alkaline phosphatase conjugate is added. The plates are then incubated, washed and alkaline phosphatase substrate is added. Color development is monitored at 405 nm, for example, on a Titertek 310 C ELISA plate reader.

The biological activity of the AAT may be determined by utilizing the ability of native AAT to bind elastase. In a preferred assay, samples containing AAT are mixed with elastase at a ratio of 1 ug elastase to 4 ug AAT. These mixtures are incubated on ice before being heated in a boiling water bath. The samples are run on a 10% acrylamide gel and transferred to nitrocellulose using the Western blotting technique (Towbin et al., Proc. Natl. Acad. Sci. USA 76:4350, 1979). Purified rabbit anti-AAT, goat anti-rabbit conjugated to horseradish peroxidase and BioRad HRP color development reagent are used to visualize the AAT samples. Samples containing active AAT will form a complex with elastase and migrate more slowly through the acrylamide gel.

Sequence analysis of the N-terminus of the recombinant protein produced by the transformed yeast cells may be used to further characterize the protein. Amino terminal sequencing may be carried out using the method described by Edman (Acta. Chem. Scand. 4:283-293, 1950). Prior to sequencing, the protein is dialyzed against ammonium bicarbonate and lyophilized to remove salts.

As will be apparent to one skilled in the art, other proteins may be purified by similar methods, using antibodies, assays and fractionation conditions appropriate to the particular protein of interest.

To summarize the examples which follow, Example 1 describes the cloning of a cDNA sequence encoding human α-1-antitrypsin. Example 2 describes the subcloning and modification of ADH2 promoter sequences. Example 3 describes the construction of the vectors pAT-2, pAT-3, pAT-4, and pAT-6, which direct the expression of α-1-antitrypsin in transformed yeast cells. Example 4 describes the transformation of yeast cells with plasmids described in Example 3. The transformants are cultured in appropriate media and produce high levels of α-1-antitrypsin. Example 5 describes methods for assaying the production of α-1-antitrypsin by transformed yeast cells. Example 6 described the cloning of a cDNA encoding the a subunit of human factor XIII. Example 7 describes the construction of factor XIII a subunit expression units. Example 8 describes the construction of expression vectors using the expression units described in Example 7 and the expression of Factor XIII a subunit in yeast. Example 9 describes a method for assaying Factor XIII activity. Example 10 describes the cloning of the cDNA encoding PAP-I. Example 11 describes the expression of PAP-I in yeast.

The following examples are offered by way of illustration and not by way of limitation.

EXAMPLES

Enzymes, including restriction enzymes, DNA polymerase I (Klenow fragment), BAL-31, T$_4$ DNA ligase, T$_4$ polynucleotide kinase, bacterial alkaline phosphatase and calf alkaline phophatase were obtain from New England Biolabs (Beverly, Mass.), Bethesda Research Laboratories (Gaithesburg, Md.) and Boerhinger-Mannheim Biochemicals (Indianapolis, Ind.) and were used as directed by the manufacturer or as described in Maniatis et al., (Molecular Cloning: A Laboratory Manual, Cold Spring Harbor, N.Y., 1982).

Oligonucleotides were synthesized on an Applied Biosystems (Foster City, Calif.) Model 380-A DNA synthesizer and purified by polyacrylamide gel electrophoresis. A human placenta cDNA library was obtained from Clontech Lab., Inc. (Palo Alto, Calif.).

EXAMPLE 1

AAT Subcloning

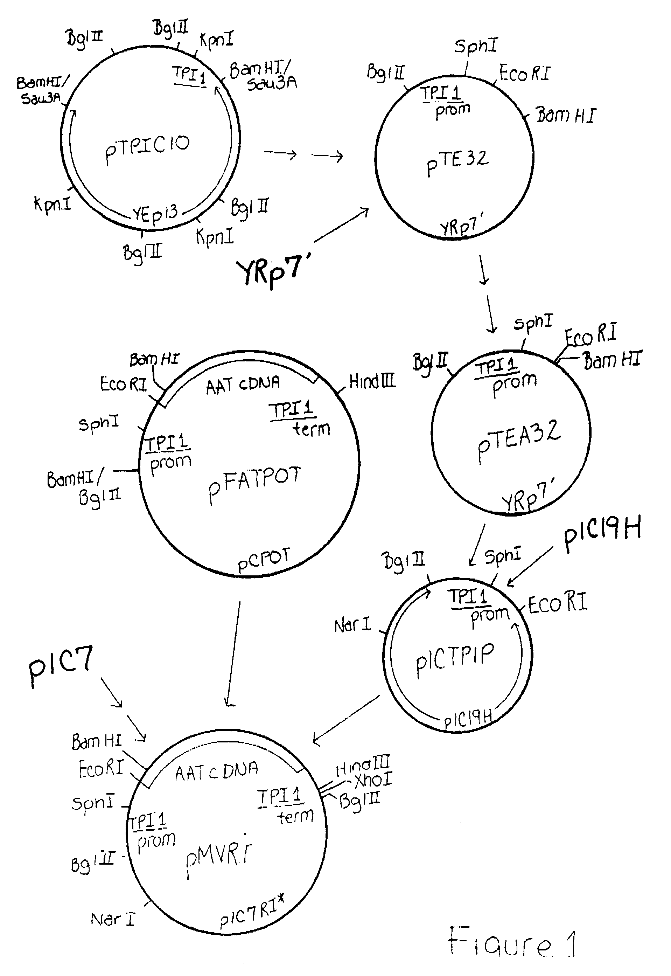

A cDNA coding for the predominant form of human α-1-antitrypsin (AAT) was isolated from a human liver cDNA library by conventional procedures using the baboon sequence (Kurachi et al., Proc. Natl. Acad. Sci. USA 78:6826-6830, 1980; and Chandra et al., Biochem. Biophys. Res. Comm. 103:751-758, 1981) as a DNA hybridization probe. The library was constructed by inserting human liver cDNA into the Pst I site of the plasmid pBR322 (Bolivar et al., Gene 2:95-113, 1977). The AAT cDNA was isolated from the library as a 1500 base pair (bp) Pst I fragment. This fragment was inserted into the Pst I site of pUC13 to produce the plasmid pUCαl. In pUCαl, the AAT sequence is flanked on the 3′ end by Xba I and Eco RI sites in the polylinker. This cDNA sequence was used to construct the plasmid pFATPOT, illustrated in Figure 1. Plasmid pFATPOT has been deposited with ATCC as a Saccharomyces cerevisiae strain E18 transformant, accession number 20699.

The AAT cDNA was then joined to the TPI1 terminator in the plasmid pMVR1. This plasmid further comprises the TPI1 promoter, and was assembled in the following manner. Plasmid pIC7 (Marsh et al., Gene 32:481-486, 1984) was digested with Eco RI, the fragment ends were blunted with DNA polymerase I (Klenow fragment), and the linear DNA was recircularized using T₄ DNA ligase. The resulting plasmid was used to transform E. coli strain RR1. Plasmid DNA was prepared from the transformants and was screened for the loss of the Eco RI site. A plasmid having the correct restriction pattern was designated pIC7RI*. The TPI1 promoter fragment was obtained from plasmid pTPIC10 (Alber and Kawasaki, ibid.), as illustrated in Figure 1. This plasmid was cut at the unique Kpn I site within the TPI1 gene, the TPI1 coding region was removed with Bal31 exonuclease, and a kinased Eco RI linker (sequence: GGAATTCC) was added to the 3′ end of the promoter. Digestion with Bgl II and Eco R1 yielded a TPI1 promoter fragment having Bgl II and Eco R1 adhesive ends. This fragment was then joined to plasmid YRp7′ (Stinchcomb et al., Nature 282:39-43, 1979) which had been cut with Bgl II and Eco RI. The resulting plasmid, TE32, was cleaved with Eco RI and Bam HI to remove a portion of the tetracycline resistance gene. The linearized plasmid was then recircularized by the addition of a kinased, annealed Eco RI-Bam HI adapter (5′ AAT TCA TGG AG 3′ and 5′ GAT CCT CCA TG 3′) to produce plasmid TEA32. Plasmid TEA32 was digested with Bgl II and Eco RI and the ⁻900 bp TPI1 promoter fragment was gel purified. Plasmid pIC19H was cut with Bgl II and Eco RI and the vector fragment was gel purified. The TPI1 promoter fragment was then ligated to the linearized pIC19H and the mixture was used to transform E. coli strain RR1. Plasmid DNA was prepared and screened for the presence of a⁻900 bp Bgl II-Eco RI fragment. A correct plasmid was selected and designated pICTPIP. Plasmic pIC7RI* was digested with Hind III and Nar I and the 2500 bp fragment was gel purified. An approximately 900 bp fragment, comprising a partial TPI1 promoter and pICl9H vector sequences, was removed from pICTPIP using Nar I and Sph I and was gel purified. Plasmid pFATPOT was digested with Sph I and Hind III and the 1750 bp fragment comprising a portion of the TPI1 promoter, an AAT cDNA, and the TPI1 terminator was gel purified. The pIC7RI* fragment, the TPI1 promoter fragment, and the TPI1 promoter-AAT-TPI1 terminator fragment from pFATPOT were then combined in a triple ligation to produce pMVR1 (Figure 1).

EXAMPLE 2

Subcloning and Modification of ADH2 Promoters

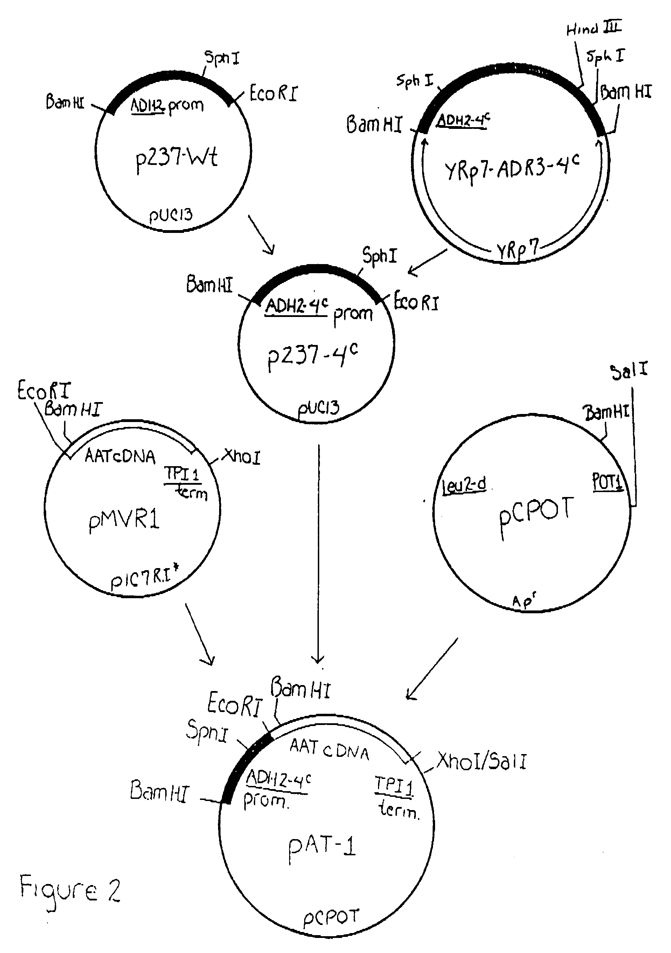

An ADH2-4ᶜ promoter was constructed by adding an Eco RI site to the 3′ end of the wild-type ADH2 promoter and combining the 3′ portion of this modified promoter with the 5′ portion of the ADH2-4ᶜ promoter. The 2.2 kb Bam HI fragment containing the wild-type ADH2 structural gene and 5′ flanking sequences from pBR322-ADR2-BSa (Williamson et al., ibid.) was ligated with M13mp19 which had been linearized with Bam HI. The orientation of the insert was determined by restriction analysis. Site-specific in vitro mutagenesis (Zoller et al., DNA 3:479-488, 1984) was done on the ADH2 insert in M13mp19 using ZC237 (Table 1) as the mutagenic primer and ZC87 (Table 1) as the second primer. In positive clones, the oligonucleotide ZC237 looped out the structural portion of the ADH2 gene, fusing the 5′ flanking sequence, including the translation start signal, with the Eco RI site of the M13mp19 polylinker. Replicative form DNA of the mutagenized phage was made and cut with Bam HT and Eco RI to isolate the 1.2 kb promoter fragment. This fragment was ligated into pUC13 which had been linearized with Bam HI and Eco RI to generate plasmid p237-Wt. To change the p237-Wt promoter to the 'promoter up' mutant $ADH2\text{-}4^c$ promoter a 1.1 kb Bam HI-Sph I fragment from YRp7-ADR3-4$^c$ (Russell et al., Nature 304:652-654, 1983), containing the alterations found to influence the promoter function, was subcloned into the vector fragment of p237-Wt which had been cut with Bam HI and Sph I. The resulting plasmid was designated p237-4$^c$ - (Figure 2).

## TABLE 1

| | |
|---|---|
| ZC87 | 5'TCC CAG TCA CGA CGT 3' |
| ZC237 | 5'GCC AGT GAA TTC CAT TGT GTA TTA 3' |
| ZC410 | 5'CGT AAT ACA GAA TTC CCG GG 3' |
| ZC411 | 5'TAA TAC ACA ATG GAG GAT CCC 3' |
| ZC862 | 5'CGA ATC TTT TGA GCT CAG AAA CAC C 3' |
| ZC1056 | 5'AAT TAG ATC TGC A 3' |
| ZC1057 | 5'GAT CT 3' |
| ZC1113 | 5'CGA CCT TCC ATG TGA TAA CTC GAG AAG CTG AGA TGA AC 3' |
| ZC1551 | 5' GAT CCC CGG GCA GCT CCT CGA GGC ATG 3' |
| ZC1552 | 5' CCT CGA GGA GCT CCC CGG G 3' |

The ADH2 promoter was then fused to the codon for the first amino acid of the mature form of AAT in the plasmid pAT-1 (Figure 2). Plasmid pAT-1 comprises the expression unit of the ADH2 promoter from p237-Wt joined to the α-1-antitrypsin cDNA-TPI1 terminator sequence from the plasmid pMVR1. These sequences were inserted into a portion of the vector pCPOT. (Plasmid pCPOT has been deposited with ATCC as an E. coli strain HB101 transformant and has been assigned accession number 39685. It comprises the entire 2 micron plasmid DNA, the leu2-d gene, pBR322 sequences and the Schizosaccaromyces pombe POT1 gene.) Plasmid pCPOT was cut with Bam HI and Sal I to isolate the approximately 10 kb linear vector fragment. pMVR1 (Example 1) was cut with Eco RI and Xho I to isolate the 1.5 kb α-1-antitrypsin cDNA-TPI1 terminator fragment. The 1.2 kb ADH2 promoter fragment was isolated from p237-Wt as a Bam HI-Eco RI fragment and was joined with the 1.5 kb α-1-antitrypsin cDNA-TPI1 terminator fragment and the linearized pCPOT in a three-part ligation to yield a plasmid designated pAT-1.

Plasmid pAT-1 contained three extra amino acid codons between the ADH2 translation start codon and the first amino acid codon for the mature form of AAT. These three codons were removed by site directed in vitro mutagenesis (Zoller et al., ibid.). Plasmid pAT-1 was cut with Sph I and Bam HI to isolate the 190 bp

EP 0 284 044 B1

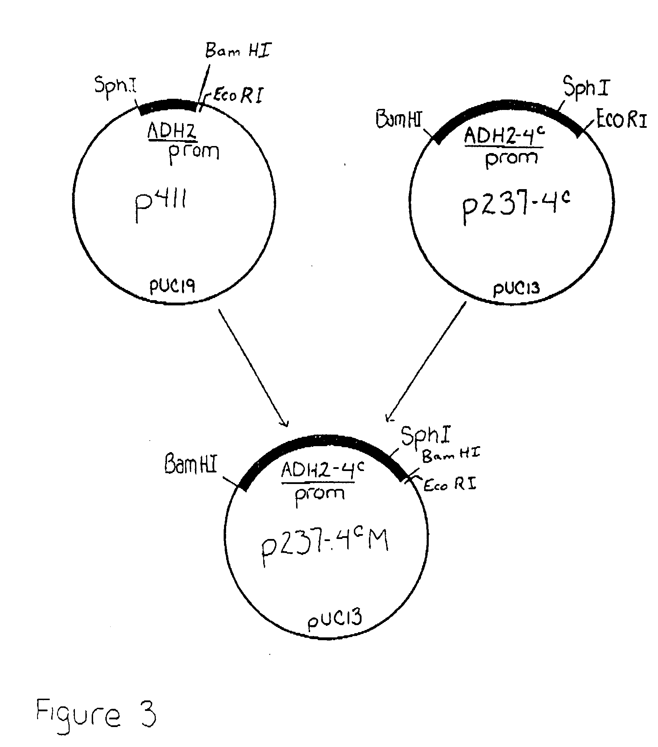

ADH2 promoter fragment. This fragment was ligated into M13mp18 which had been linearized with Bam HI and Sph I. The resulting construction was subjected to in vitro mutagenesis using ZC411 (Table 1) as the mutagenic primer and ZC87 as the second primer to fuse the ADH2 translation start signal to the first codon of mature $\alpha$-1-antitrypsin. Positive clones were confirmed by dideoxy sequencing from -170 bp from the ATG through the fusion point. For ease of manipulation, the 175 bp Sph I-Eco RI mutagenized promoter fragment was ligated into pUC19 linearized with Sph I and Eco RI. The resultant plasmid, comprising the 3′ most 170 bp of the ADH2 promoter and the ADH2 translation start fused to the first amino acid of the mature form of AAT in vector pUC19, was designated p411 (Figure 3).

To generate the complete ADH2-4$^c$ promoter fused to the first amino acid of mature AAT, the 5′ most sequence of the ADH2-4$^c$ promoter, containing the alterations found by Russell et al. (ibid.) to influence promoter function, was added to the promoter fragment present in plasmid p411 (Figure 3). Plasmid p411 was digested with Sph I and Eco RI to isolate the 175 bp promoter fragment. Plasmid p237-4$^c$ was cut with Eco RI and Sph I to isolate the 3.71 kb fragment comprising the vector sequences and the 5′ most promoter sequence that confers the "promoter-up" phenotype. The 175 bp promoter fragment from p411 was ligated into the p237-4$^c$ vector fragment. The resulting plasmid, containing the complete ADH2-4$^c$ promoter fused to the first amino acid codon of the mature AAT sequence, was designated p237-4$^c$M.

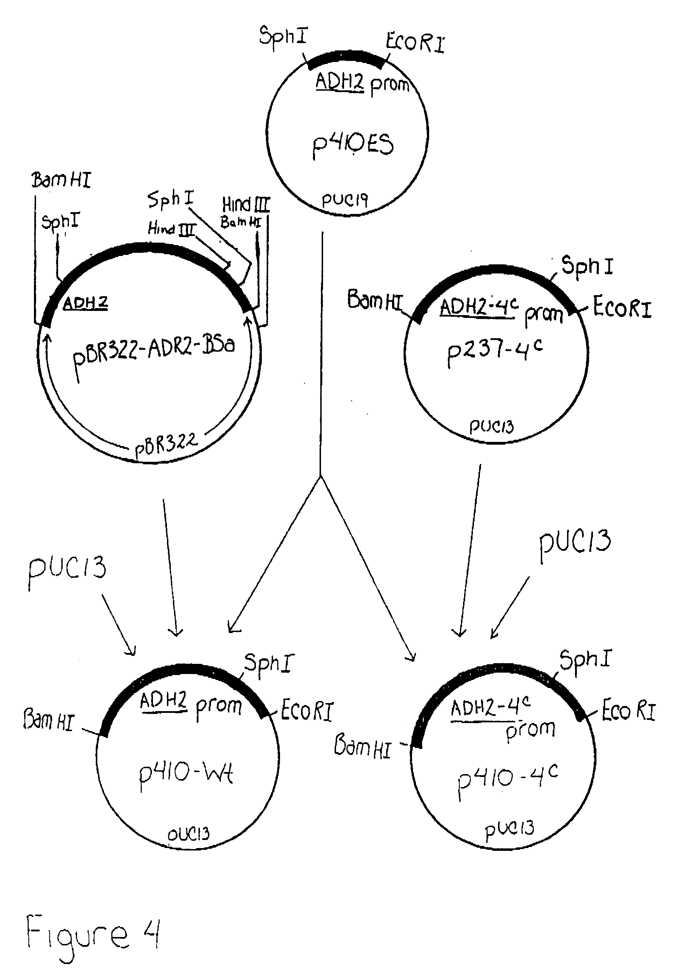

The ADH2 promoter from plasmid pAT-1 was modified to create a "universal" promoter by removing the ADH2 translation start site and the pUC18 polylinker sequences found in pAT-1 (Figure 4). Plasmid pAT-1 was cut with Sph I and Bam HI to isolate the 190 bp partial ADH2 promoter fragment. This fragment was ligated into M13mp18 linearized with Bam HI and Sph I. The resulting construction was subjected to in vitro mutagenesis (Zoller et al., ibid.) using ZC410 (Table 1) as the mutagenic primer and ZC87 as the second primer. The mutagenesis using ZC410 replaces the ADH2 translation start signal and pUC18 polylinker sequences with a single Eco RI site fused to the M13mp18 polylinker at the Sma I site. Positive clones were confirmed by dideoxy sequencing through the fusion point. For ease of manipulation, the mutagenized partial ADH2 promoter fragment was subcloned as a 175 bp Sph I-Eco RI fragment into pUC19 which had been linearized by Sph I and Eco RI. The resulting plasmid, designated p410ES, contained the 3′most 175 bp of the ADH2 promoter. The wild-type ADH2 promoter was regenerated using the partial ADH2 promoter fragment from p410ES. Plasmid p410ES was digested with Sph I and Eco RI to isolate the 175 bp partial ADH2 promoter fragment. This fragment was joined with a 1 kb Bam HI-Sph I fragment derived from pBR322-ADR2-BSa in a three-part ligation into pUC13 which had been linearized by digestion with Bam HI and Eco RI. The 1 kb fragment derived from pBR322-ADR2-BSa contained sequences that are homologous with wild-type ADH2 promoter sequence. The plasmid that resulted from the three-part ligation was confirmed by restriction analysis and designated p410-Wt.

A universal ADH2-4$^c$ promoter was generated using the mutagenized promoter fragment from plasmid p410ES (Figure 4). The 1.1 kb fragment containing sequences known to confer the ADH2$^c$ promoter phenotype was taken from plasmid p237-4$^c$. Plasmid p237-4$^c$ was cut with Bam HI and Sph I to isolate the 1.1 kb ADH2-4$^c$ promoter fragment. The ADH2-4$^c$ promoter was reconstructed in a three way ligation which joined the Bam HI-Sph I promoter fragment from p237-4$^c$, the Sph I-Eco RI mutagenized promoter fragment from p410ES, and pUC13 which had been linearized with Bam HI and Eco RI. The resultant plasmid, confirmed by restriction analysis, contained the complete ADH2-4$^c$ promoter mutagenized at the 3′ end to place an Eco RI site in place of the translation start codon. The plasmid was designated p410-4$^c$.

The "universal" promoter from plasmid p410-4$^c$ was used to replace the TPI1 promoter present in plasmid pMVR1 (Example 1). Plasmid p410-4$^c$ was cut with Bam HI and Eco RI to isolate the 1.2 kb ADH2-4$^c$ promoter fragment. Plasmid pMVR1 was digested to completion with Eco RI and partially digested with Bgl II to isolate the 4.2 kb AAT-TPI1 terminator-vector fragment. These two fragments were ligated to form the plasmid designated pTRK4c.

EXAMPLE 3

Construction of Expression Vectors pAT-2, pAT-3, pAT-4 and pAT-6

A. Construction of pAT-2

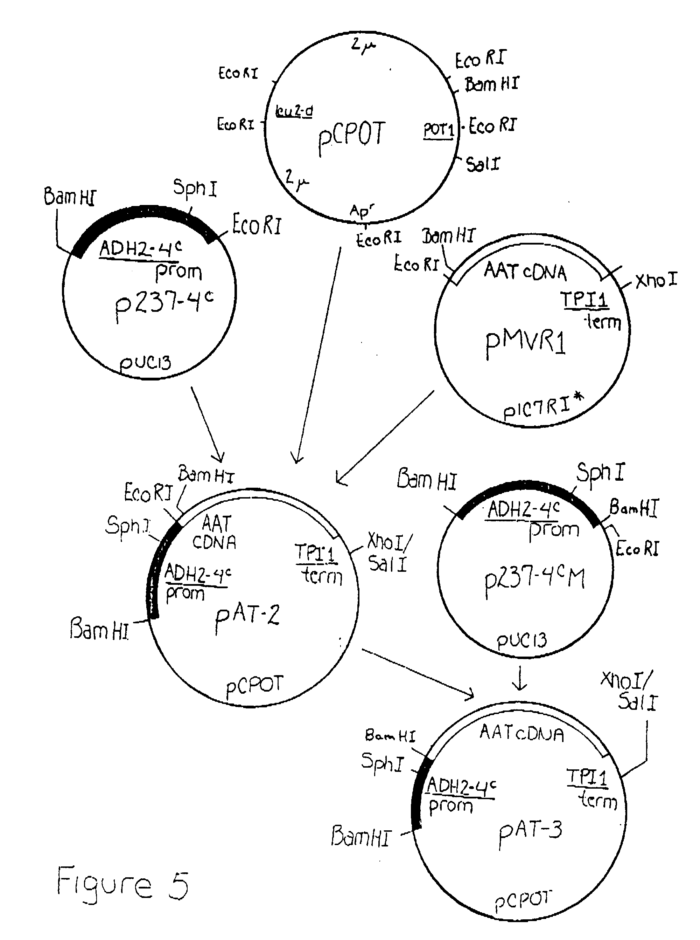

The expression vector pAT-2, comprising the ADH2-4$^c$ promoter, an AAT cDNA and the TPI1 terminator was assembled as follows (Figure 5). Plasmid pCPOT was digested to completion with Bam HI and Sal I to remove the POT1 gene. The 10 kb linear vector fragment was isolated. The ADH2-4$^c$ promoter fragment was taken from plasmid p237-4$^c$. Plasmid p237-4$^c$ was cut with Bam HI and Eco RI to isolate the 1.2 kb ADH2-4$^c$ promoter fragment containing the complete promoter with an Eco RI site 3′ to the translation start codon. The AAT cDNA-TPI1 terminator fragment was isolated as a 1.5 kb Eco RI-Xho I fragment from plasmid pMVR1. These three fragments were joined in a three-part ligation and transformed into E. coli strain RR1. The resultant plasmids were analyzed by restriction analysis. The correct plasmid, designated pAT-2, comprises the ADH2-4$^c$ promoter, the AAT cDNA and the TPI1 terminator in the vector pCPOT.

B. Construction of pAT-3

The expression unit in plasmid pAT-2 encodes three extra amino acids between the ADH2 translation start and the first amino acid of the mature form of AAT. These three amino acids were removed by replacing the ADH2-4$^c$ promoter present in plasmid pAT-2 with the ADH2-4$^c$ promoter from plasmid p237-4$^c$M (Figure 5). Plasmid pAT 2 was cut with Bam HI to isolate the 12.4 kb vector fragment comprising the α-1-antitrypsin cDNA, the TPI1 terminator and the vector pCPOT. This fragment was treated with calf alkaline phosphatase to prevent recircularization upon ligation. Plasmid p237-4$^c$M was digested with Bam HI to isolate the 1.2 kb fragment comprising the ADH2-4$^c$ promoter fused to the ATC and first codon of the AAT cDNA. This fragment was ligated into the linearized fragment from pAT-2. The orientation of the ADH2-4$^c$ promoter was determined by restriction analysis and the plasmid with the promoter in the correct orientation was designated pAT-3. Plasmid pAT-3, comprising the ADH2-4$^c$ promoter fused directly to the AAT cDNA, the TPI1 terminator and the vector pCPOT, utilizes the leu2-d selection system to achieve high plasmid copy number when transformed into appropriate strains of S. cerevisiae. S. cerevisiae strain ZM103 transformed with pAT-3 (transformant designated ZM110) has been deposited with the American Type Culture Collection.

C. Construction of plasmid pAT-4.

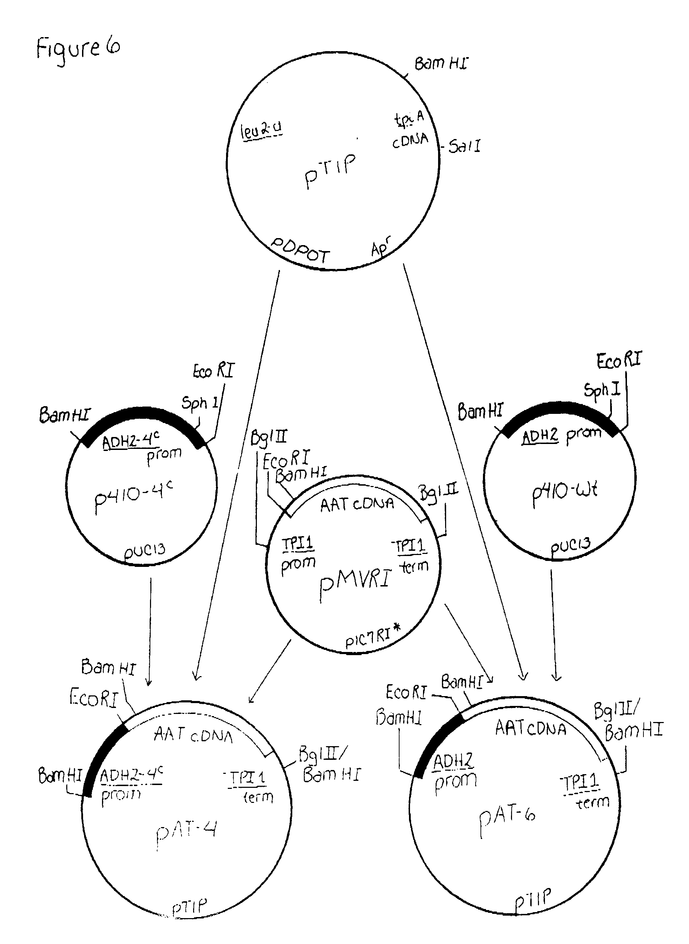

The ADH2-4$^c$ promoter-AAT cDNA expression unit was placed in the vector pTIP to generate plasmid pAT-4 (Figure 6). The vector, pTIP, comprises the A. nidulans tpiA cDNA (McKnight et al., Cell 46:143-147, 1986) linked to the E. coli lacZ promoter, which can complement the tpil mutation in S. cerevisiae. It also contains the leu2-d selectable marker. The A. nidulans tpiA cDNA was subcloned in pUC19 (McKnight et al., ibid.) and was re-isolated as a 1.2 kb Bam HI-partial Sst I fragment. This fragment was ligated into pIC19R that has been linearized by digestion with Sst I and Bgl II. The resultant plasmid, pM144, was linearized with Eco RV and ligated to kinased Bam HI linkers. The linear fragment was then cut with Sal I and Bam HI to remove excess linkers and to isolate the 1.2 kb tpiA cDNA. This fragment was ligated with pIC19R that had been linearized by digestion with Sal I and Bam HI. The resultant plasmid, pM147, was the source for the tpiA cDNA used in pTIP. Plasmid pM147 was cut with Sal I and Bam HI to isolate the 1.2 kb tpiA cDNA. Plasmid pCPOT was altered by replacing the 750 bp Sph I-Bam HI fragment containing 2 micron and pBR322 sequences with a 186 bp Sph I-Bam HI fragment derived from the pBR322 tetracyline resistance gene. The resulting plasmid, designated pDPOT, was cleaved with Bam HI and Sal I to remove the S. pombe POT1 gene and to isolate the 10.2 kb vector fragment. These two fragments were ligated together, resulting in the plasmid designated pTIP. Plasmid pTIP comprises the origin of replication, REP1, REP2 and REP3 of 2 micron plasmid, pBR322 sequences encoding the E. coli Amp$^R$ gene, the Col E1 origin of replication and the defective selectable markers leu2-d and tpiA, allowing selection for high plasmid copy number using either the leu2-d selection system or the tpiA selection system or both.

Plasmid pAT-4 was then assembled as follows (Figure 6). Plasmid pTIP was linearized by digestion with Bam HI and treated with bacterial alkaline phosphatase to prevent recircularization. Plasmic pMVR1 was digested with Eco RI and Bgl II to isolate the 1.5 kb α-1-antitrypsin cDNA-TPI1 terminator fragment. The universal ADH2-4$^c$ promoter from plasmid p410-4$^c$ was isolated as a 1.2 kb Bam HI-Eco RI fragment. This fragment was joined with the 1.5 kb α-1-antitrypsin cDNA-TPI1 terminator fragment into the linearized pTIP vector in a three-part ligation. The ligation mixture was transformed into E. coli strain RRI. Plasmid DNA made from the transformants was analyzed by restriction analysis to identify a positive clone. This clone was designated pAT-4.

D. Construction of plasmid pAT-6.

The wild-type ADH2 promoter was fused to the α-1-antitrypsin cDNA and placed in the vector pTIP to obtain plasmid pAT-6. Plasmid pTIP was linearized with Bam HI and treated with bacterial alkaline phosphatase to prevent recircularization. Plasmid pMVR1 was digested with Bgl II and Eco RI to isolate the 1.5 kb α-1-antitrypsin cDNA-TPI1 terminator fragment. The universal ADH2 promoter was isolated from plasmid p410-Wt as a 1.2 kb Bam HI-Eco RI fragment. This fragment was joined with the 1.5 kb α-1-antitrypsin cDNA-TPI1 terminator fragment into the linearized pTIP vector in a three-part ligation. The ligation mixture was transformed into E. coli strain RRI. Plasmid DNA made from the transformants was analyzed by restriction analysis to identify a positive clone. The clone was designated pAT-6.

As a result of the cloning strategy used, the promoter sequences present in both pAT-4 and pAT-6 deviate from the wild-type sequence immediately 5′ to the ATG. These sequence alterations result in sequences around the initiation codon which may lead to reduced expression levels. In order to maximize expression levels, these plasmids are digested with Bam HI to remove the promoter, ATG and first codon of mature AAT. A promoter fragment comprising a precise ADH2-AAT fusion is then substituted. In the case of pAT-4, plasmid p237-4ᶜM is digested with Bam HI and the ADH2-4ᶜ promoter fragment is isolated. The promoter is then joined to the Bam HI-cut pAT-4 and a plasmid having the correct promoter orientation is selected.

EXAMPLE 4

Transformation of Host Cells and Expression of Alpha-1-Antitrypsin

The plasmids pAT-3 and pAT-4 were transformed into suitable yeast hosts utilizing methods well known in the art (Beggs, J.D., ibid., 1978); Hinnen et al., Proc. Natl. Acad. Sci. USA 75:1929-1933, 1978). The S. cerevisiae strains utilized were genotypically pep4 and had been cured of endogenous 2 micron plasmid, rendering them [cir°]. Additionally, the strains contained mutations complemented by the defective selectable markers present on the plasmids.

Plasmid pAT-3, comprising the ADH2-4ᶜ promoter fused to the AAT cDNA in the vector pCPOT, was transformed into S. cerevisiae strain ZM103 (MATa leu2-3,112 ura3 pep4::URA3 bar1 gal2 [cir°]. Transformants were selected for their ability to grow on -LEUDS (Table 2).

## TABLE 2

### -LEUDS plates

20 g Glucose

6.7 g Yeast Nitrogen Base without Amino Acids (DIFCO Laboratories)

40 mg adenine

30 mg L-arginine-HCl

50 mg L-aspartic acid

20 mg L-histidine free base

60 mg L-isoleucine

40 mg L-lysine-monohydrochloride

20 mg L-methionine

60 mg L-phenylalanine

50 mg L-serine

50 mg L-tyrosine

40 mg uracil

60 mg L-valine

182 g sorbitol

18 g agar (DIFCO Laboratories)

Mix all ingredients in distilled water. Add distilled water to a final volume of 1 liter. Autoclave 15 minutes. After autoclaving, add 150 mg L-threonine and 40 mg L-tryptophan. Pour plates and allow to solidify.

### -LEUD

Use the recipe for -LEUDS plates, omitting sorbitol and agar. Autoclave for 15 minutes and add the L-threonine and L-tryptophan after removal of the media from the autoclave.

### MED A

20 g Yeast Extract (DIFCO Laboratories)

5 g ammonium sulfate (Sigma, St. Louis, Mo.)

10 ml 100% Ethanol

Mix yeast extract and ammonium sulfate in distilled water. Add distilled water to a final volume of 1 liter. Autoclave 25 minutes. After autoclaving, cool media and add 10 ml 100% EtOH.

### -LEU 1% EtOH

Use the recipe for -LEUD omitting the glucose. Mix all ingredients in distilled water. Add distilled water to a final volume of 1 liter. Autoclave 15 minutes. After autoclaving, add 150 mg L-threonine and 40 mg L-tryptophan. Cool media and add 10 ml 100% ethanol.

-URADS plates

20 g Glucose

6.7 g Yeast Nitrogen Base without Amino Acids (DIFCO Laboratories, Detroit, Mich.)

40 mg adenine

30 mg L-arginine-HCl

50 mg L-aspartic acid

20 mg L-histidine free base

60 mg L-isoleucine

80 mg L-leucine

40 mg L-lysine-monohydrochloride

20 mg L-methionine

60 mg L-phenylalanine

50 mg L-serine

50 mg L-tyrosine

60 mg L-valine

182.2 g sorbitol

18 g agar (DIFCO Laboratories)

Mix all ingredients in distilled water. Add distilled water to a final volume of 1 liter. Autoclave 15 minutes. After autoclaving, add 150 mg L-threonine and 40 mg L-tryptophan. Pour plates and allow to solidify.

-URAD

Use the recipe for -URADS plates, omitting the sorbitol and agar. Autoclave for 15 minutes and add the L-threonine and L-tryptophan after removal of the media from autoclave.

-TRPDS

20 g Glucose

6.7 g Yeast Nitrogen Base without Amino Acids (DIFCO)

40 mg adenine

30 mg L-arginine

50 mg L-aspartic acid

14

20 mg L-histidine free base

60 mg L-isoleucine

80 mg L-leucine

40 mg L-lysine-monohydrochloride

20 mg L-methionine

60 mg L-phenylalanine

50 mg L-serine

50 mg L-tyrosine

40 mg uracil

60 mg L-valine

182.2 g sorbitol

18 g agar (DIFCO)

Mix all ingredients in distilled water. Add distilled water to a final volume of 1 liter. Autoclave 15 minutes. After autoclaving, add 150 mg L-threonine. Pour plates and allow to solidify.

YEPD

20 g glucose

10 g yeast extract (DIFCO)

20 g peptone (DIFCO)

Mix all ingredients in distilled water. Add distilled water to a final volume of 1 liter. Autoclave 25 minutes.

Expression of AAT from the pAT-3 plasmid in the strain ZM103 was achieved by first growing transformants overnight at 30°C in 10 ml of -LEUD (Table 2). This allows the plasmid to achieve and maintain high copy number by selection for the weak complementation of the leu2-d gene located on pAT-3. The cells were pelleted and the spent medium was discarded. AAT expression was induced by resuspending the cells in MED A (Table 2) and growing the cells at 30°C for 48 hours. The ethanol present in MED A as the sole carbon source depresses the ADH2-4$^c$ promoter, allowing for increased expression of AAT. Using this method, AAT was detected at 30% of total cell protein.

Plasmid pAT-4, comprising the ADH2-4$^c$ promoter fused to the AAT cDNA in the vector pTIP, was transformed into S. cerevisiae strain ZM115 (MATa ade2-101 leu2-3,112 ura3 gal2 Δpep4::TPI1-CAT tpiI::URA3 [cir°]). Transformants were selected for their ability to grow on -LEUDS.

Expression of AAT from the pAT-4 plasmid in the strain ZM115 was achieved by first growing the transformants overnight at 30°C in 25 ml -LEUD. This allows the plasmid to achieve and maintain high copy number by using the double selection for weak complementation of the leu2-d gene and the lacZ-tpiA expression unit located on pAT-4. AAT expression was induced by diluting the culture 1:50 in 25 ml -LEU 1% EtOH (Table 2) at 30°C for 40 hours. The ethanol present in the media as the sole carbon source derepresses the ADH2-4$^c$ promoter, allowing for increased expression of AAT.

15

EXAMPLE 5

Description of AAT Assays

A. Enzyme-Linked Immunosorbant Assay (ELISA)

Appropriately grown cells, as described in the previous section, were centrifuged to pellet the cells and the spent media were discarded. The cells were pelleted and washed with distilled water. The washed pellets were frozen at -80°C before being assayed.

Crude glass bead lysates were made of the frozen cell pellets. The washed cell pellets were thawed on ice and diluted in an equal volume of phosphate-buffered saline (PBS, Sigma, St. Louis, Mo.). Glass beads (450-500 um) were added to one half the total volume. This mixture was vortexed at full speed for one minute, three times, with the samples cooled on ice between vortex bursts. Larger samples of 50 ml or more were treated the same way and lysed in a Bead Beater (Biospec Products, Bartlesville, Okla). The larger volumes were cooled in an ethanol ice bath between vortex bursts. The liquid was removed from the tubes with a pasteur pipet and transferred to a microfuge tube. The glass beads were washed one time in the original volume of PBS. The beads were vortexed one minute and the liquid was removed by pasteur pipet and pooled with the original lysate. The lysates were then centrifuged in an Eppendorf microfuge (Brinkmann, Westbury, N.Y.) at top speed for five minutes. The supernatant was carefully removed for assay.

The double antibody ELISA was initiated by first adhering antibody to the wells of a microtiter plate. Rabbit anti-$\alpha$-1-antitrypsin (Calbiochem) that had been affinity purified over a human $\alpha$-1-antitrypsin column was diluted in 0.1 M $Na_2CO_3$, pH 9.7 to a concentration of 5 ug/ml. 500 ng of antibody were added to each well and the mixtures were incubated overnight at 4°C. Excess antibody was removed by two washes with buffer C (PBS + 0.5% Tween 20 + 0.05% $NaN_3$ + 1% bovine serum albumin). Excess bovine serum albumin (BSA) was removed by two washes with buffer C. Standards and samples were diluted in buffer B in duplicate. 100 l of each sample were added to the wells and incubated at 37°C for one hour. Unbound material was removed by three washes with buffer C. Biotin-conjugated mouse monoclonal anti-$\alpha$-1-antitrypsin Fab was diluted to 1 ug/ml in buffer B. 100 ul of the biotin conjugated mouse anti-$\alpha$-1-antitrypsin Fab were added to each well and incubated at 37°C for one hour. Excess Fab was removed by three washes with buffer C. Avidin-alkaline phosphatase (Boehringer Mannheim, Indianapolis, Indiana) was diluted 1:2500 in buffer B and 100 ul were added to each well. The mixtures were incubated at 37°C for one hour, with the excess avidin-alkaline phosphatase being washed off with three washes of buffer C at the end of the hour. Sigma Phosphatase Substrate was diluted to 0.6 mg/ml in buffer D (1 liter distilled water containing 96 mls diethanolamine + 56 mg $MgCl_2$ and pH adjusted pH 9.8). 100 ul of substrate were added to each well and incubated at room temperature for 15-30 min. or until appropriate color formation. The absorbance at 405 nm of the supernatant was measured and the concentration was determined relative to a standard curve. The total soluble protein concentration of the yeast lysates was determined by the procedure of Lowry et al. (ibid.). The amount of $\alpha$-1-antitrypsin was expressed as a percentage of total soluble protein.

B. Biological Activity Assay

Elastase binding assays were performed on the samples to qualitatively demonstrate $\alpha$-1-antitrypsin activity. Samples containing $\alpha$-1-antitrypsin as determined by ELISA were mixed with elastase at a ratio of 1 ug elastase: 4 ug $\alpha$-1-antitrypsin (as determined by ELISA) in a total of 50 ul TNE (0.02 M Tris pH 8, 0.05 M NaCl, 1 mM EDTA). These mixtures were incubated for 45 min. on ice, then heated in a boiling water bath for 3 min. The samples were run on a 10% acrylamide gel and transferred to nitrocellulose using the method described by Towbin (Proc. Natl. Acad. Sci. USA 76:4350, 1979). Purified rabbit anti-$\alpha$-1-antitrypsin, goat anti-rabbit conjugated to horse radish peroxidase and BioRad HRP Color Development Reagent were used to visualize the $\alpha$-1-antitrypsin samples. Samples containing active $\alpha$-1-antitrypsin will form a complex with elastase and migrate more slowly across the 10% polyacrylamide gel.

EXAMPLE 6

Cloning of a cDNA Encoding the a Subunit of Human Factor XIII

A. Screening for cDNA Encoding the a Subunit of Human Factor XIII.

A λgt11 expression library containing cDNAs prepared from human placenta mRNA was screened for the a subunit of human Factor XIII. An $^{125}$I-labeled affinity-purified rabbit antibody (specific activity = 6 X $10^6$ cpm/ug) was used to screen filters containing phage plated at a density of 1.5 X $10^5$ plaques per 150 mm plate. Six positive clones were isolated by screening approximately 3 X $10^6$ phage, and each positive phage was plaque-purified.

Plaque-purified cones were then screened with the following $^{32}$p-labeled oligonucleotide probe: $^5$'CTC CAC GGT GGG CAG GTC GTC CTC G$^3$'. This probe codes for the amino acid sequence of Ala-Glu-Asp-Asp-Leu-Pro-Thr-Val-Glu that is present in the activation peptide of the a subunit (Takagi and Doolittle, Biochem. J. 13:750-756, 1974). The nucleotide sequence for the probe was selected by employing the most common codon usage for amino acids for a number of different human proteins (Chen and Barker, Trends in Genetics 1:221-223, 1985). The oligonucleotide was labeled with $^{32}$p to a specific activity of 1.1 X $10^8$ cpm/ug.

B. DNA Sequencing of cDNA Inserts

Phage DNA was prepare from positive clones by the liquid culture lysis method (T.J. Silhavy et al., in Experiments with Gene Fusions, CSH Laboratory, N.Y., pp. 140-141, 1984), followed by centrifugation and banding on a cesium chloride step gradient. cDNA inserts were isolated by digestion of the phage DNA with Eco RI endonuclease and the 5$^′$ and 3$^′$ ends of each insert were sequenced. One of the clones with a large cDNA insert (λHFXIIIa3.77, ATCC No. 40261) was selected for further sequence analysis. This insert contained three internal Eco RI sites, giving rise to four cDNA fragments upon digestion of the phage DNA with Eco RI. These fragments and several additional restriction fragments were subcloned into plasmid pUC9 or pUC19. Additional restriction fragments from other cDNA inserts were subcloned into M13mp10, M13mp11, m13mp18, or M13mp19 in order to obtain overlapping sequences. The cDNA inserts were then sequenced by the dideoxy method (Sanger et al., Proc. Natl. Acad. Sci. USA 74:5463-5467, 1977) using [α$^{35}$S]dATP and buffer gradient gels (Biggin et al., Proc. Natl. Acad. Sci. USA 80:3963-3965, 1983). Digestions with nuclease BAL-31 were performed to generate five additional fragments that provided overlapping sequences with the Eco RI restriction fragments.

The sequence of 3831 base pairs from these overlapping clones codes for the entire amino acid sequence of the mature a subunit of human Factor XIII that circulates in blood. The a subunit is composed of 731 amino acids, starting with an amino- terminal sequence of Ser-Glu-Thr-Ser. This amino-terminal sequence was reported earlier by Takagi and Doolittle (ibid.). The carboxyl-terminal Met (nucleotides 2281-2283) is followed by a stop codon (TGA), 1535 base pairs of noncoding sequence, and a potential polyadenylation or processing signal of AATAAA. The polyadenylation sequence was located 14 nucleotides upstream from the poly(A) tail of 10 nucleotides. The poly(A) tail was present only in a second cDNA clone, designated λHFXIIIa3.82.

The cDNA insert in λHFXIIIa3.77 also codes for 30 amino acid residues that appear to encode an in-frame sequence. Cleavage of the bond between Met and Ser to yield mature Factor XIII would require an additional processing protease(s) to yield the mature protein with an amino-terminal Ser. This would be followed by an acetylation reaction, leading to the formation of acetyl-serine at the amino-terminal end of the mature protein (Takagi and Doolittle, ibid.; Nakamura eta al., J. Biochem. 78:1247-1266, 1975).

Differences in the nucleotide sequence for the a subunit of Factor XIII were found at three positions when a comparison of the cDNA inserts was made in regions where overlapping sequences were obtained. Nucleotides 2038, 2041, and 2727 contained A, C, and T, respectively, in λHFXIIIa3.77, while λHFXIIIa3.82 contained G. C, and A in the same positions. These differences result in a change in two amino acids (Ile 650 and Gln 651 to Val and Glu), and could represent a polymorphism that contributes to the microheterogeneity in the a subunit of Factor XIII (Board and Coggan, Hum. Genet. 59:135-136, 1981).

EXAMPLE 7

Construction of Factor XIII a Subunit Expression Units

A. Construction of pRS202

The Factor XIII a subunit (asFXIII) cDNA was modified by in vitro mutagenesis to replace the 3′ noncoding region with an Xho I site. For ease of manipulation, the 3.8 kb asFXIII cDNA insert contained in phage clone λHFXIIIa3.82 was subcloned into pUC18. The phage clone λHFXIIIa3.82 was digested to completion with Pst I to isolate the 2.3 kb fragment comprising the asFXIII cDNA. This fragment was ligated with pUC18 which had been linearized by digestion with Pst I. The resultant plasmid, pUC18 #9, was determined to have the 2.3 kb Pst I insert in the anti-sense orientation. The asFXIII cDNA insert present in pUC18 #9 comprised 19 bp 5′ to the translation start, the asFXIII coding region and 120 bp 3′ to the translation stop.

The asFXIII cDNA insert was then isolated and subcloned into the vector pUC118 (obtained from J. Vieira and J. Messing, Waksman Institute of Microbiology, Rutgers, Piscataway, N.J.). The 2.3 kb asFXIII insert was isolated from pUC18 #9 by digestion with Pst 1, subcloned into the pUC118 Pst I site and transformed into E. coli strain JM109. A positive clone, which contained the 2.3 kb asFXIII insert in the anti-sense orientation, was designated pRS201.

The 120 bp 3′ untranslated region of the asFXIII cDNA was removed and an Xho I site was inserted 3′ to the translation stop codon by site directed mutagenesis. Plasmid pRS201 was transformed into E. coli strain MV1193 and single-stranded template DNA was isolated. Oligonucleotide ZC1113 (Table 1) was designed to remove the 3′ untranslated region following the asFXIII coding sequence and to introduce an Xho I site 3′ to the translation stop. The single-stranded template of pRS201 was subjected to in vitro mutagenesis by the method of Zoller et al., (ibid., 1984) using the mutagenic oligonucleotide ZC1113. A positive clone, confirmed by restriction analysis, was designated pRS202.

B. Construction of Plasmid pRS215

The 2.2 kb asFXIII cDNA fragment from plasmid pRS202 was placed behind the ADH2-4$^c$ promoter and placed in plasmid pIC7RI*. Plasmid pTRK4c (Example 2) was digested to completion with Eco RI and Sal I to isolate the 4 kb fragment comprising the ADH2-4$^c$ promoter, the TPI1 terminator and the pIC7RI* vector sequences. Oligonucleotides ZC1056 and ZC1057 (Table 1) were designed to form an adapter with an Eco RI adhesive end, a Bgl II site and a Pst I adhesive end. The Eco RI adhesive end of the adapter, upon ligation to another Eco RI adhesive end, destroys the Eco RI site. Oligonucleotides ZC1056 and ZC1057 were kinased and annealed to form the Eco RI adapter. The 2.2 kb Pst I-Xho I asFXIII fragment, isolated from plasmid pRS202 was joined with the ZC1056/ZC1057 adapter and the 4 kb pTRK4c fragment in a three-part ligation. The resultant plasmid was designated pRS215.

EXAMPLE 8

Expression of Factor XIII a Subunit in Yeast

A. Construction of the Vector pEAS102

Plasmid pEAS102, comprising portions of the vectors YIp5 and pJDB207, was constructed as follows. Plasmid pJDB207 (Beggs, Proceedings of Alfred Benzon Symposium 16:383-389, "Molecular Genetics in Yeast," Copenhagen, Denmark, 1981), a derivative of pJDB219 (J.D. Beggs, Nature 275:104-108, 1978) was digested with Bam HI and Pst I to isolate the 4.4 kb fragment comprising the leu2-d gene, 2 micron DNA and pBR322 sequences. Plasmid YIp5 (Struhl et al., Proc. Natl. Acad. Sci. USA 76:1035-1039, 1979) was subjected to partial digestion with Pst I and complete digestion with Bam HI to isolate 4.3 kb fragment comprising the URA3 gene and pBR322 sequences. These two fragments were ligated and the resultant plasmid was designated pEAS102.

B. Construction of Plasmid pRS217

A yeast expression vector comprising the ADH2-4C promoter, an asFXIII cDNA, the TPI1 terminator and pEAS102 vector sequences was constructed. Plasmid pRS217 was constructed as follows. The expression

18

unit present in pRS215 (Example 7) was placed in the yeast vector pEAS102. Plasmid pEAS102 was digested to completion with Hind III followed by treatment with bacterial alkaline phosphatase to prevent recircularization. Plasmid pRS215 was digested with Hind III to isolate the 3.5 kb expression unit comprising the ADH2-4$^c$ promoter, an asFXIII cDNA and the TPI1 terminator. These two fragments were ligated together to create plasmid pRS217.

C. Construction of the Vector pRPOT

Plasmid pDPOT (Example 3) was modified by the insertion of a polylinker. Plasmid pDPOT was digested with Sph I and Bam HI to isolated the 10.8 kb fragment. Oligonucleotides ZC1551 and ZC1552 (Table 1) were designed to form an adapter with a Bam HI adhesive end and an Sph I adhesive end flanking Sma I, Sst I and Xho I restriction sites. Oligonucleotides ZC1551 and ZC1552 were kinased and annealed to form the Bam HI-Sph I adapter. The 10.8 kb pDPOT fragment was circularized by ligation with the ZC1551/ZC1552 adapter. The resultant plasmid was termed pRPOT.

D. Construction of Plasmid pJRXIII

The expression unit from plasmid pRS215, comprising the TPI1 promoter, an asFXIII cDNA and the TPI1 terminator, was placed in the yeast/ E. coli shuttle vector pRPOT. Plasmid pJRXIII, comprising the expression unit from pRS215 and pRPOT vector sequences was constructed as follows. Plasmid pRS215 was digested with Xho I to isolate the 3.5 kb expression unit. The pRS215 expression unit was joined by ligation with pRPOT which had been linearized by digestion with Xho I and subsequently treated with calf alkaline phosphatase to prevent self-ligation. The ligation was transformed into E. coli strain MC1061 (Dagert and Ehrlich, Gene 6:23-28, 1979). Plasmid DNA isolated from the transformants was analyzed by restriction analysis. A positive clone was identified which contained the expression unit in the same orientation as the POT1 gene and was designated pJRXIII.

E. Construction of Plasmids pD15 and pD16

The expression unit in plasmid pRS215 was also inserted into the yeast/E. coli shuttle vector pDPOT. Plasmid pDPOT was linearized by digestion with Bam HI followed by treatment with calf alkaline phosphatase to prevent recircularization. Plasmid pRS215 was digested with Sst I and Pst I to isolate the 0.65 kb fragment comprising a portion of the ADH2-4$^c$ promoter and the ZC1056/ZC1057 adapter. Plasmid pRS215 was also digested with Pst I and Bgl II to isolate the 2.3 kb fragment comprising the asFXIII cDNA and TPI1 terminator. Plasmid p410-4$^c$ (Example 2) was digested with Bam HI and Sst I to isolate the 0.55 kb fragment comprising a 5$^\prime$ portion of the ADH2-4$^c$ promoter. The 0.55 kb Bam HI-Sst I fragment derived from p410-4$^c$, the 0.65 kb Sst I-Pst I fragment and 3.1 kb Pst I-Bgl II fragments from pRS215 were joined with the Bam HI linearized pDPOT in a four-part ligation. Two plasmids were identified as having the correct insert in opposite orientations. Plasmid pD15 contained the expression unit with the ADH2-4$^C$ prompter distal to the POT1 gene in the vector. Plasmid pD16 contained the expression unit with the ADH2-4$^c$ promoter proximal to the POT1 gene in the vector.

F. Transformation of pRS217, pJRX111, and pD16 and the Expression of Active Factor XIII in Yeast

Plasmid pRS217 was transformed into Saccharomyces cerevisiae strain XV794-7-1C (MATa ade2-1 leu2-3 leu2-112 ura3 Δpep4::TPI1-CAT [cir$^+$] using the method essentially described by Beggs (Nature275:104-108, 1978). Transformants were selected for the ability to grow on -URADS plates (Table 2).

Expression of the Factor XIII as subunit from the pRS217 plasmid transformed into the strain XV794-7-1C was achieved by first growing the transformants overnight in 5 ml -URAD (Table 2). The overnight cultures were inoculated into 1 liter -URAD and grown for 48 hours at 30°C. The cultures were centrifuged to harvest the cell pellets, which were washed once with distilled water and stored at -80°C.

Plasmids pJRXIII and pD16 were transformed into the yeast host strain ZM118 (MATa/MATα [cir°] homozygous for Δtpil::URA3, pep4::URA3, leu2-3,112, ura3, and bar1) and selected on synthetic medium lacking tryptophan and supplemented with 1 M sorbitol (-TRPDS plates, Table 2).

Expression of active Factor XIII from pJRXIII and pD16 transformed into strain ZM118 was achieved by first growing the transformants overnight in 5 ml YEPD (Table 2). Two ml of the overnight culture was inoculated into 200 ml YEPD and grown at 30°C with shaking. The cultures were centrifuged to harvest the cell pellets, which were washed once with distilled water and stored at -80°C.

The cell pellets were assayed as described in Example 9. All the transformants were found to produce active Factor XIII. However, a comparison of the transformants showed that pJRXIII transformants produced 6 times the active Factor XIII than pRS217 transformants produced and pD16 transformants produced 25 times the active Factor XIII that pRS217 transformants produced.

EXAMPLE 9

Description of Factor XIII Assays

A. Preparation of Cell Pellets

Crude glass bead lysates were made from the frozen cell pellets. The washed cell pellets were thawed on ice and diluted in an equal volume of phosphate buffered saline (PBS; Sigma, St. Louis, Mo.), 1 mM $\beta$-mercaptoethanol (Sigma). Glass beads (450-500 um) were added to one half the total volume. This mixture was vortexed at full speed for one minute, three times, with the samples cooled on ice between vortex bursts. The liquid was removed from the tubes with a pasteur pipet and transferred to a microfuge tube. The glass beads were washed one time in the original volume of PBS containing 1 mM $\beta$-mercaptoethanol (BME). The beads were vortexed one minute and the liquid was removed by pasteur pipet and pooled with the original lysate. The lysates were then centrifuged in an Eppendorf microfuge (Brinkmann, Westbury, N.Y.) at top speed for five minutes. The supernatant was carefully removed for assay.

B. Factor XIII Activity Assay

Factor XIII activity was measured using the method essentially described by Curtis and Lorand (Methods Enzymol. 45:177-191, 1976). The lysates were measured for total yeast protein by the method described by Lowry et al. (J. Biol. Chem. 193:265-275, 1951). Samples were diluted to 10 ug/ul total yeast protein with PBS + 1 mM BME. Standards using commercially available Factor XIII (Table 3) were diluted in stepwise dilution in 50 mM Tris-HCl pH7.5, 10 mM dithiothreitol. Five ul of sample or standard were added to 35 ul 50 mM Tris-HCl pH 7.6 and 1 ul thrombin (Table 3). The mixtures were allowed to incubate at 37°C for 30 minutes. The reactions were quenched by the addition of 2 U Hirudin (Sigma). Sixty ul Reaction Mixture (Table 3) 37°C was added and the mixture was incubated for 30 minutes. The reactions were stopped by adding 1 ml 7.5% trichloroacetic acid (TCA). The samples were spun in the microfuge at top speed for 5 minutes and the supernatants were discarded. The pellets were washed twice with 7.5% TCA. The pellets were then dissolved in 100 ul glacial acetic acid. Five ml scintillation cocktail (Optifluor, Packard Instruments Co., Downers Grove, Ill.) were added and the samples were counted on the scintillation counter (Beckman, Palo Alto, Calif.).

## TABLE 3

| Thrombin: | Lyophilized thrombin (Sigma, St. Louis, Mo.) is dissolved in 50% Glycerol, .25 M Tris-HCl pH 7.5 to 1000 U/ml. This solution is stored at -20°C. |
|---|---|
| Commercial Factor XIII: | Factor XIII (Green Cross, Osaka, Japan) dissolved in 1 ml. 50% Glycerol, 50 mM Tris-HCl pH 7.5, 10 mM dithiothreitol (DTT; Sigma, St. Louis, Mo.). This solution is stored at -20°C. |
| N,N,Dimethylcasein: | N,N,Dimethylcasein (Sigma, St. Louis, Mo.) is dissolved to 10 mg/ml in 0.5 M Tris-HCl pH7.5. |
| $^3$H-Histamine Dihydrochloride: | 1 mCi $^3$H-Histamine dihydrochloride (New England Nuclear, Boston Mass.) was dissolved in 4 ml 10 mM unlabeled histamine dihydrochloride. This solution is stored at -20°C. |
| Reaction Buffer: | 1.4 M NaCl<br>1.0 M Tris Base<br>0.1 M CaCl$_2$<br>0.2 M DTT<br>pH adjusted to 7.5 |
| Reaction Mixture: | 2.0 ml $^3$H-Histamine dihydrochloride<br>8.0 ml N,N,Dimethylcasein<br>1.8 ml Reaction Buffer |

EXAMPLE 10

Cloning of cDNA Encoding PAP-I

Isolation and characterization of the anticoagulant protein is disclosed by Funakoshi et al. (Biochem. J. 26:5572-5578, 1987). A human placenta cDNA library (Clontech) was screened using affinity-purified antibody against PAP-I according to the methods of Young and Davis (Proc. Natl. Acad. Sci. USA 80: 1194-1198, 1983) and Foster and Davie Proc. Natl. Acad. Sci. USA 81: 4766-4770, 1984). Twelve positive clones were obtained from 5 X 10⁵ recombinants and were then plaque-purified. Sequence analysis of the largest clone (1.5 kb insert) showed that this clone contained an open reading frame sequence coding for PAP-I starting from residue 38 and extending to the 3′ non-coding region containing the poly(A) tail. The original library was then re-screened using this clone as a hybridization probe. The probe was labeled by the method of Maniatis et al. (Proc. Natl. Acad. Sci. USA 72:1184-1188, 1975). Filters were washed with 2 X SSC buffer (8.2 g of Na-citrate pH 7.0 and 17.5 g of NaCl/liter) containing 0.5% SDS at 60°C for 1 hour. Twenty-four clones were then obtained and plaque-purified. Positive clones were subcloned into M13mp18 or M13mp19 for sequence analysis using the dideoxy-³⁵S method of Sanger et al. (Proc. Natl. Acad. Sci. USA 74: 5463-5467, 1977). The largest clone (1.7 kb insert) was found to encode a nearly full length cDNA and included an initiation Met residue at the 5′ end followed by the entire mature protein, a stop codon, and a polyadenylation signal. The cDNA sequence of PAP-I does not contain a leader peptide sequence, indicating that PAP-I is probably not constitutively secreted. The presence of Met at the 5′ end by cDNA

21

EP 0 284 044 B1

sequence analysis and the absence of this Met by protein sequence analysis showed that the Met residue is removed in a post-translational event and the newly formed $NH_2$-terminal Ala residue is blocked by acetylation.

EXAMPLE 11

Expression of PAP-I in Yeast

For expression in yeast, the PAP-I cDNA was linked to the ADH2-4c promoter and the TPI1 terminator. This expression unit was inserted into several yeast expression vectors and the vectors were used to transform selected yeast strains.

The PAP-I cDNA was joined to the ADH2-4c promoter as follows. Plasmid pAP1.7, comprising the 1.7 kb cDNA in pUC18, was cut with Nco I and Bam HI and the linearized plasmid was isolated through two rounds of gel purification. The ADH2-4c promoter was functionally linked to the 5′ end of the PAP-I cDNA through an adaptor having the following structure:

AATTCTACAC
GATGTGGTAC

This was achieved by a three-part ligation using Nco I, Bam HI cut vector, the 1.2 kb Bam HI-Eco RI promoter fragment from p410-4c (Example 2) and the adaptor. The resultant plasmid was designed pPR1. The presence of the correct promoter fusion was confirmed by DNA sequencing.

Expression vectors were then constructed. Plasmid pZUC13 (pZUC13 comprises the S. cerevisiae chromosomal LEU2 gene and the origin of replication from the S. cerevisiae 2 micron plasmid inserted into pUC13. It constructed in a manner analogous to pZUC12, described in published European Patent Application 195,691, using the plasmid pMT212, which is described in published European Patent Application 163,529) was cut with Sal I and treated with calf intestinal phosphatase to prevent self-ligation. Plasmid pPR1 was digested completely with Sal I and Bgl II and the -2.4 kb promoter + PAP-I fragment was recovered. The yeast TPI1 terminator fragment was obtained from plasmid pFG1 (Alber and Kawasaki, J. Mol. Appl. Genet. 1: 419-434, 1982). It encompasses the region from the penultimate amino acid codon of the TPI1 gene to the Eco RI site approximately 700 base pairs downstream. A Bam HI site was substituted for this unique Eco RI site of pFGI by first cutting the plasmid with Eco RI, then blunting the ends with DNA polymerase I (Klenow fragment), adding synthetic Bam HI linkers (CGGATCCA), and re-ligating to produce plasmid p136. The TPI1 terminator was then excised from p136 as a Xba I-Bam HI fragment. This fragment was ligated into YEp13 (Broach et al., Gene 8: 121, 1979) which had been linearized with Xba I and Bam HI. The resulting plasmid is known as p213. The Hind III site was then removed from the TPI1 terminator region of p213 by digesting the plasmid with Hind III, blunting the resultant termini with DNA polymerase I (Klenow fragment), and recircularizing the linear molecule using $T_4$ DNA ligase. The resulting plasmid was designated p270.

Alternatively, p270 may be constructed by digesting plasmid pM220 (deposited with American Type Culture Collection as an E. coli RR1 transformant, accession number 39853) with Xba I and Bam HI, purifying the TPI1 terminator fragment (-700 bp) and inserting this fragment into Xba I, Bam HI digested YEp13.

The TPI1 terminator was removed from plasmid p270 as a Xba I-Bam HI fragment. This fragment was cloned into pUC19 along with another fragment containing the TPI1 promoter fused to the CAT (chloramphenicol acetyl transferase) gene to obtain a TPI1 terminator fragment with an Eco RV end. The resultant plasmid was designated pCAT (Figure 11). The TPI1 terminator was then cut from pCAT as an Eco RV-Bam HI fragment and cloned into pIC19H (Marsh et al., ibid.) which had been cut with the same enzymes, to obtain pTT1. Plasmid pTT1 was digested with Sal I and Bgl II and the -800 bp TPI1 terminator fragment was recovered. The three fragments (Sal I-cut pZUC13, Sal I- Bgl II promoter + PAP-I and Bgl II-Sal I terminator) were then joined. Two different expression vectors with opposite expression unit orientations were obtained. These vectors were designated pZ1 and pZ2.

The expression unit from pZ2 was inserted into the yeast/E. coli shuttle vector pDPOT. Plasmid pZ2 was cut with Bam HI to isolate the 3.2 kb expression unit. Plasmid pDPOT was linearized by digestion and was subsequently treated with calf alkaline phosphatase to prevent recircularization. The 3.2 kb expression unit was joined with the linearized pDPOT fragment by ligation. Two different expression vectors were isolated with opposite orientations. These vectors were designated pD1 and pD2. Plasmid pD2 contained

22

the expression unit with the direction of transcription away from the POT1 gene.

Plasmid pZ2 was transformed into the S. cerevisiae strain ZA521 (MATa leu2-3, 112 ura3 pep4::URA3 bar1 gal2 [cir°]). The transformants were grown in 10 ml of -LEUD (Table 1) overnight at 30°C and the optical densities of the cultures were determined at 600 nm. The cell pellets were resuspended in MED A to an optical density equalling 8 at 600 nm. The cultures were incubated with shaking for 28 hours. Plasmid pD2 was transformed into S. cerevesiae strain ZM118. The transformants were grown in YEPD (Table 2) for 96 hours at 30°C.

The cultures were centrifuged to pellet the cells and the spent medium was discarded. The cells were pelleted and washed with distilled water. The washed pellets were frozen at -80°C before being assayed.

Crude glass bead lysates were made of the frozen cell pellets. The washed cell pellets were thawed on ice and diluted in an equal volume of phosphate-buffered saline (PRS). Glass beads (450-500 um) were added to one half the total volume. The cells were lysed by vortexing the mixture at full speed for one minute, three times, with the samples cooled on ice between vortex bursts. The liquid was removed from the tubes with a pasteur pipet and transferred to a microfuge tube. The glass beads were washed once in the original volume of PBS. The beads were vortexed one minute and the liquid was removed by pasteur pipet and pooled with the original lysate. The lysates were then centrifuged in an Eppendorf microfuge (Brinkmann, Westbury, N.Y.) at top speed for five minutes. The supernatants were carefully removed and assayed for total soluble protein by the method described by Lowry et al. (J. Biol. Chem. 193:265-275, 1951).

The lysates were subsequently electrophoresed on polyacrylamide gels. Approximately 50 ug of total soluble protein as assayed by the Lowry method (Lowry et al., ibid.) were loaded on a 12% polyacrylamide gel along with PAP-I standards of 1, 2 or 5 ug of purified PAP-I. The sample was electrophoresed and visualized by coomasie blue staining. By comparison of the yeast produce PAP-I with the purified PAP-I standard, it was found that the PAP-I produced by pZ2 transformants represented approximately 2% of the total soluble protein compared with the pD2 transformants which produced PAP-I at approximately 20% of the total soluble protein.

From the foregoing it will be appreciated that, although specific embodiments of the invention have been described herein for purposes of illustration, various modifications may be made without deviating from the spirit and scope of the invention. Accordingly, the invention is not limited except as by the appended claims.

The features disclosed in the foregoing description, in the claims and/or in the accompanying drawings may, both separately and in any combination thereof, be material for realising the invention in diverse forms thereof.

## Claims

1. An expression vector capable of directing the expression of heterologous genes or cDNA in yeast, said expression vector containing an ADH2 promoter, a heterologous gene or cDNA, and a defective selectable marker.

2. The expression vector of claim 1 wherein said ADH2 promoter is a mutant isolate of the ADH2 promoter.

3. The expression vector of claim 1 wherein said ADH2 promoter is the ADH2-4$^c$ promoter.

4. The expression vector of claim 1 wherein said vector further includes functional genetic elements derived from a yeast 2 micron circle.

5. The expression vector of claim 4 wherein said elements include an origin of replication, and REP1, REP2 and REP3 genes.

6. The expression vector of claim 1 wherein said vector includes the entire yeast 2 micron circle.

7. The expression vector of claim 1 wherein said defective selective marker comprises the Schizosaccharomyces pombe POT1 gene, Aspergillus nidulans tpiA cDNA operably linked to the E. coli lacZ promoter, or the leu2-d gene.

**8.** The expression vector of claim 1 wherein said defective selectable marker comprises a mutant structural gene.

**9.** The expression vector of claim 1 wherein said defective selectable marker includes a deletion or alteration in a promoter operably linked to said marker.

**10.** The expression vector of claim 1 wherein said heterologous gene or cDNA encodes $\alpha$-1-antitrypsin, factor XIII or PAP-I.

**11.** A yeast host cell having a genetic defect into which has been introduced an expression vector according to any of claims 1-10, wherein the defective selectable marker of the expression vector is complementary to the genetic defect of the yeast host cell.

**12.** The yeast host cell of claim 11 wherein said yeast cell is a strain of S. cerevisiae.

**13.** A method for the enhanced production of proteins in yeast host cells comprising:
introducing into a yeast host cell having a genetic defect an expression vector according to any of claims 1-10, wherein the defective selectable marker of the expression vector is complementary to the genetic defect of the yeast host cell.
growing said yeast host cell in an appropriate medium; and
isolating the protein produced by said host cell.

**14.** A method for the enhanced production of $\alpha$-1-antitrypsin in yeast host cells, comprising:
introducing into a [cir°] strain of S. cerevisiae auxotrophic for growth on leucine, and carrying a pep4 mutation, an expression vector capable of directing the expression of heterologous genes or cDNA in yeast including an origin of replication, and REP1, REP2 and REP3 genes derived from a yeast 2 micron circle, said expression vector containing an ADH2-4$^c$ promoter, a gene or cDNA encoding $\alpha$-1-antitrypsin, and a defective selectable marker consisting of a leu2-d gene;
growing said yeast host cells in an appropriate medium;
disrupting said yeast host cells; and
isolating the $\alpha$-1-antitrypsin produced by said yeast host cells.


**Patentansprüche**

**1.** Ein Expressionsvektor, der in der Lage ist, die Expression von heterologen Genen oder heterologer cDNA in Hefe zu steuern, wobei besagter Expressionsvektor einen ADH2-Promotor, ein heterologes Gen oder eine heterologe cDNA und einen unvollkommenen selektionierbaren Marker enthält.

**2.** Der Expressionsvektor nach Anspruch 1, wobei besagter ADH2-Promotor ein mutantes Isolat des ADH2-Promotors ist.

**3.** Der Expressionsvektor nach Anspruch 1, wobei besagter ADH2-Promotor der ADH2-4$^c$ -Promotor ist.

**4.** Der Expressionsvektor nach Anspruch 1, wobei besagter Vektor außerdem funktionale genetische Elemente einschließt, die abgeleitet sind von einem Hefe-2-Mikron-Kreis.

**5.** Der Expressionsvektor nach Anspruch 4, wobei besagte Elemente einen Replikationsursprung und REP1-, REP2- und REP3-Gene einschließen.

**6.** Der Expressionsvektor nach Anspruch 1, wobei besagter Vektor den gesamten Hefe-2-Mikron-Kreis einschließt.

**7.** Der Expressionsvektor nach Anspruch 1, wobei besagter unvollkommener selektiver Marker das Schizosaccharomyces pombe-POT1-Gen, die Aspergillus nidulans-tpiA-cDNA, operabel verknüpft mit dem E. coli-lacZ-Promotor, oder das leu2-d-Gen umfasst.

**8.** Der Expressionsvektor nach Anspruch 1, wobei besagter unvollkommener selektionierbarer Marker ein mutantes Struktur-Gen umfasst.

**9.** Der Expressionsvektor nach Anspruch 1, wobei besagter unvollkommener selektionierbarer Marker eine Deletion oder Alteration in einem mit besagtem Marker operabel verknüpften Promotor einschließt.

**10.** Der Expressionsvektor nach Anspruch 1, wobei besagtes heterologes Gen oder besagte heterologe cDNA α-1-Antitrypsin, Faktor XIII oder PAP-I kodiert.

**11.** Eine Hefe-Wirtszelle mit einem genetischen Defekt, in die ein Expressionvektor nach einem der Ansprüche 1-10 eingeführt worden ist, wobei der unvollkommene selektionierbare Marker des Expressionsvektors komplementär zum genetischen Defekt der Hefe-Wirtszelle ist.

**12.** Die Hefe-Wirtszelle nach Anspruch 11, wobei besagte Hefezelle ein Stamm von S. cerevisiae ist.

**13.** Ein Verfahren zur gesteigerten Produktion von Proteinen in Hefe-Wirtszellen, welches umfasst:
Einführen eines Expressionsvektors nach einem der Ansprüche 1-10 in eine Hefe-Wirtszelle mit einem genetischen Defekt, wobei der unvollkommene selektionierbare Marker des Expressionsvektors komplementär zum genetischen Defekt der Hefe-Wirtszelle ist;
Züchten besagter Hefe-Wirtszelle in einem geeigneten Medium; und
Isolieren des von besagter Wirtszelle produzierten Proteins.

**14.** Ein Verfahren zur gesteigerten Produktion von α-1-Antitrypsin in Hefe-Wirtszellen, welches umfasst:
Einführen eines Expressionsvektors, der in der Lage ist, die Expression von heterologen Genen oder heterologer cDNA in Hefe zu steuern, einschließlich eines Replikationsursprungs und REP1-, REP2- und REP3-Genen, abgeleitet von einem Hefe-2-Mikron-Kreis, in einen [cir⁰]-Stamm von S. cerevisiae, der für Wachstum auf Leucin auxotroph ist und eine pep4-Mutation trägt, wobei besagter Expressionsvektor einen ADH2-4ᶜ-Promotor, ein Gen oder eine cDNA, die α-1-Antitrypsin kodiert, und einen unvollkommenen selektionierbaren Marker, der aus einem leu2-d-Gen besteht, umfasst;
Züchten besagter Hefe-Wirtszellen in einem geeigneten Medium;
Aufbrechen besagter Hefe-Wirtszellen; und
Isolieren des von besagten Hefe-Wirtszellen produzierten α-1-Antitrypsins.

**Revendications**

**1.** Vecteur d'expression capable de diriger l'expression de gènes ou d'ADNc hétérologues dans une levure, ledit vecteur d'expression contenant un promoteur ADH2, un gène ou ADNc hétérologue et un marqueur sélectionnable défectif.

**2.** Vecteur d'expression suivant la revendication 1, dans lequel le promoteur ADH2 est un isolat mutant du promoteur ADH2.

**3.** Vecteur d'expression suivant la revendication 1, dans lequel le promoteur ADH2 est le promoteur ADH2-4ᶜ.

**4.** Vecteur d'expression suivant la revendication 1, dans lequel le vecteur comprend en outre des éléments génétiques fonctionnels dérivés d'un élément circulaire de 2 micromètres de levure.

**5.** Vecteur d'expression suivant la revendication 4, dans lequel les éléments comprennent une origine de réplication et les gènes REP1, REP2 et REP3.

**6.** Vecteur d'expression suivant la revendication 1, qui comprend la totalité de l'élément circulaire de 2 micromètres de levure.

**7.** Vecteur d'expression suivant la revendication 1, dans lequel le marqueur sélectionnable défectif comprend le gène POT1 de Schizosaccharomyces pombe, l'ADNc tpiA d'Aspergillus nidulans lié de manière fonctionnelle au promoteur lacZ de E. coli, ou le gène leu2-d.

**8.** Vecteur d'expression suivant la revendication 1, dans lequel le marqueur sélectionnable défectif comprend un gène structural mutant.

25

**9.** Vecteur d'expression suivant la revendication 1, dans lequel le marqueur sélectionnable défectif comprend une délétion ou modification dans un promoteur lié de manière fonctionnelle audit marqueur.

**10.** Vecteur d'expression suivant la revendication 1, dans lequel le gène ou l'ADNc hétérologue code pour l'$\alpha$-1-antitrypsine, le facteur XIII ou PAP-I.

**11.** Cellule hôte de levure possédant une déficience génétique, dans laquelle a été introduit un vecteur d'expression suivant l'une quelconque des revendications 1 à 10, le marqueur sélectionnable défectif du vecteur d'expression étant complémentaire de la déficience génétique de la cellule hôte de levure.

**12.** Cellule hôte de levure suivant la revendication 11, qui est une souche de S. cerevisiae.

**13.** Procédé de production accrue de protéines dans des cellules hôtes de levures, comprenant :
l'introduction dans une cellule hôte de levure possédant une déficience génétique d'un vecteur d'expression suivant l'une quelconque des revendications 1 à 10 ; le marqueur sélectionnable défectif du vecteur d'expression étant complémentaire de la déficience génétique de la cellule hôte de levure ;
la culture de ladite cellule hôte de levure dans un milieu approprié ; et
l'isolement de la protéine produite par ladite cellule hôte.

**14.** Procédé de production accrue d'$\alpha$-1-antitrypsine dans des cellules hôtes de levures, comprenant :
l'introduction dans une souche [cir °] de S. cerevisiae auxotrophe pour la croissance sur la leucine, et portant une mutation pep4, d'un vecteur d'expression capable de diriger l'expression de gènes ou d'ADNc hétérologues dans une levure, comprenant une origine de réplication et les gènes REP1, REP2 et REP3 dérivés d'un élément circulaire de 2 micromètres de levure, ledit vecteur d'expression contenant un promoteur ADH2-4$^c$, un gène ou ADNc codant pour l'$\alpha$-1-antitrypsine, et un marqueur sélectionnable défectif consistant en un gène leu2-d ;
la culture desdites cellules hôtes de levure dans un milieu approprié ;
la rupture desdites cellules hôtes de levure ; et
l'isolement de l'$\alpha$-1-antitrypsine produite par lesdites cellules hôtes de levure.

Figure 1

Figure 2

Figure 3

Figure 4

Figure 5

Figure 6